(19) Europäisches Patentamt
European Patent Office
Office européen des brevets

(11) **EP 3 657 299 A1**

(12) **EUROPEAN PATENT APPLICATION**
published in accordance with Art. 153(4) EPC

(43) Date of publication:
**27.05.2020 Bulletin 2020/22**

(21) Application number: **18835149.8**

(22) Date of filing: **09.07.2018**

(51) Int Cl.:
*G06F 3/01* (2006.01)   *A61B 3/113* (2006.01)
*G02B 27/02* (2006.01)   *G06F 3/038* (2013.01)
*G09G 5/00* (2006.01)   *H04N 5/64* (2006.01)

(86) International application number:
**PCT/JP2018/025843**

(87) International publication number:
**WO 2019/017224 (24.01.2019 Gazette 2019/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.07.2017 JP 2017139286**

(71) Applicant: **Sony Corporation**
**Tokyo 108-0075 (JP)**

(72) Inventor: **NOMURA, Eisuke**
**Tokyo 108-0075 (JP)**

(74) Representative: **MFG Patentanwälte**
**Meyer-Wildhagen Meggle-Freund**
**Gerhard PartG mbB**
**Amalienstraße 62**
**80799 München (DE)**

(54) **INFORMATION PROCESSING DEVICE, INFORMATION PROCESSING METHOD AND PROGRAM**

(57)   An information processing device according to the present technology includes a controller. The controller is configured to acquire first position-and-pose information related to a position and a pose of hardware that is used to calibrate a line of sight, and second position-and-pose information related to a position and a pose of the hardware, the second position-and-pose information being different from the first position-and-pose information; to control a display section to display a plurality of presented gaze points including a first presented gaze point and a second presented gaze point, the first presented gaze point being presented at a first position, the second presented gaze point being presented at a second position different from the first position; to control an image-capturing section to acquire a first image of an eyeball of a user gazing at the first presented gaze point, and a first image of the eyeball of the user gazing at the second presented gaze point; to generate a plurality of pieces of calibration data using the first position-and-pose information, the second position-and-pose information, and the first images; and to perform a correction related to an optical-axis vector and a visual-axis vector with respect to the eyeball of the user, using the plurality of pieces of calibration data.

FIG.12

**EP 3 657 299 A1**

**Description**

Technical Field

The present technology relates to an information processing device that includes a line-of-sight detection device, an information processing method, and a program.

Background Art

In recent years, a technology has been developed that calibrates a line of sight of a user before detecting the line of sight (for example, refer to Patent Literature 1). The calibration includes instructing a user to direct his or her line of sight in a specified direction, and acquiring a state of an eye of the user, the eye being an eye for which the line of sight is adjusted according to the instruction. Accordingly, it becomes possible to perform correction with respect to a result of detecting a line of sight of a user according to a state of his or her eye that is acquired upon performing calibration.

Citation List

Patent Literature

Patent Literature 1: Japanese Patent Application Laid-open No. 2009-183473

Disclosure of Invention

Technical Problem

An object of the present technology is to provide an information processing device, an information processing method, and a program that make it possible to improve a performance in a detection of a line of sight.

Solution to Problem

An information processing device according to an aspect of the present technology includes a controller. The controller is configured to acquire first position-and-pose information related to a position and a pose of hardware that is used to calibrate a line of sight, and second position-and-pose information related to a position and a pose of the hardware, the second position-and-pose information being different from the first position-and-pose information; to control a display section to display a plurality of presented gaze points including a first presented gaze point and a second presented gaze point, the first presented gaze point being presented at a first position, the second presented gaze point being presented at a second position different from the first position; to control an image-capturing section to acquire a first image of an eyeball of a user gazing at the first presented gaze point, and a first image of the eyeball of the user gazing at the second presented gaze point; to generate a plurality of pieces of calibration data using the first position-and-pose information, the second position-and-pose information, and the first images; and to perform a correction related to an optical-axis vector and a visual-axis vector with respect to the eyeball of the user, using the plurality of pieces of calibration data.

Advantageous Effects of Invention

As described above, the present technology makes it possible to improve a performance in a detection of a line of sight. Brief Description of Drawings
[Fig. 1] Fig. 1 illustrates a configuration of an information processing device 100 according to a first embodiment of the present technology.
[Fig. 2] Fig. 2 conceptually illustrates a configuration of a line-of-sight detection device 10 included in the information processing device 100 illustrated in Fig. 1.
[Fig. 3] Fig. 3 is a block diagram of a hardware configuration of the line-of-sight detection device 10 illustrated in Fig. 2.
[Fig. 4A] Fig. 4A illustrates an actual relationship between an optical-axis vector $V_{opt} \rightarrow$ and a visual-axis vector $V_{vis} \rightarrow$ when an image-capturing section 12 in a typical line-of-sight detection device is arranged offset in an optical-axis direction y of the image-capturing section 12.
[Fig. 4B] Fig. 4B illustrates a calculated relationship between the optical-axis vector $V_{opt} \rightarrow$ and the visual-axis vector

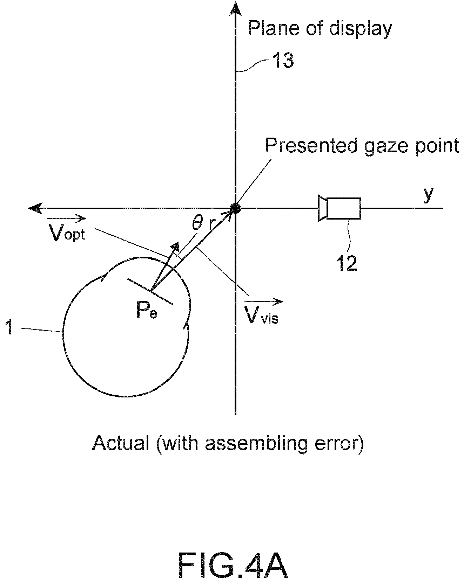

$V_{vis}\rightarrow$ replacing the relationship illustrated in Fig. 4A, the calculated relationship being obtained by performing a line-of-sight calibration.

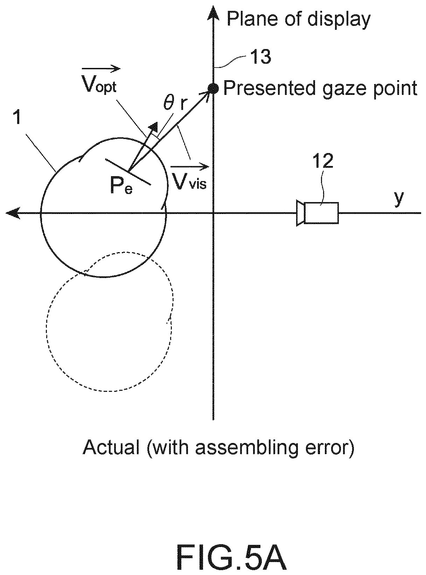

[Fig. 5A] Fig. 5A illustrates an actual relationship between an optical-axis vector $V_{opt}\rightarrow$ and a visual-axis vector $V_{vis}\rightarrow$ when the position of a line-of-sight-estimation-target eyeball 1 of a user moves in a direction orthogonal to an optical-axis direction y of the image-capturing section 12 with respect to a position upon performing a line-of-sight calibration, in which there is an assembling error in the image-capturing section 12 with respect to the optical-axis direction y.

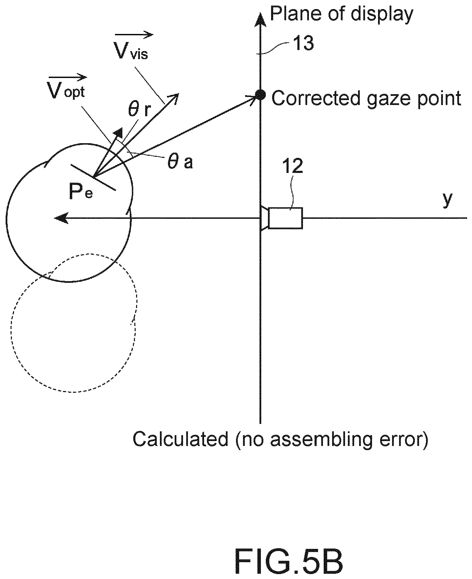

[Fig. 5B] Fig. 5B illustrates a calculated relationship between the optical-axis vector $V_{opt}\rightarrow$ and the visual-axis vector $V_{vis}\rightarrow$ replacing the relationship illustrated in Fig. 5A, the calculated relationship being obtained by performing the line-of-sight calibration.

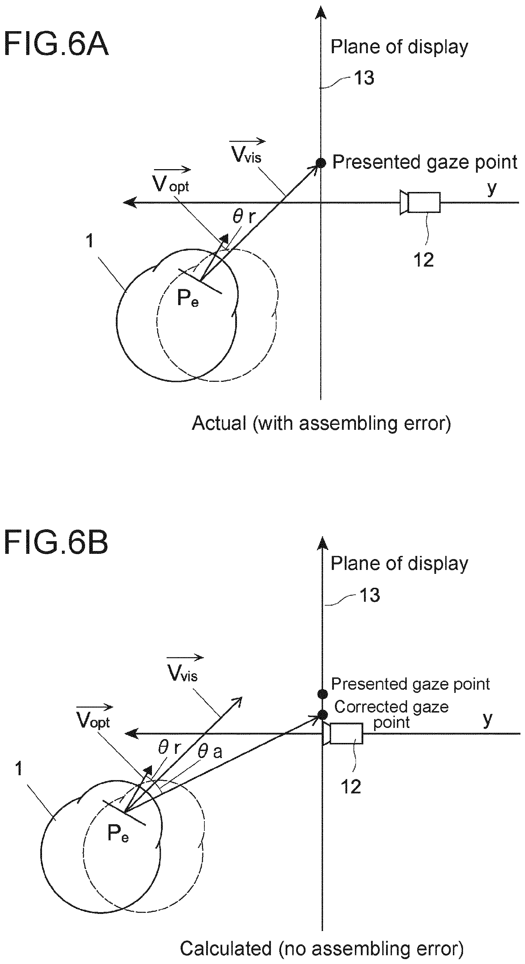

[Fig. 6A] Fig. 6A illustrates an actual relationship between an optical-axis vector $V_{opt}\rightarrow$ and a visual-axis vector $V_{vis}\rightarrow$ when the position of the line-of-sight-estimation-target eyeball 1 of the user moves in a direction other than the direction orthogonal to the optical-axis direction y of the image-capturing section 12 with respect to the position upon performing a line-of-sight calibration.

[Fig. 6B] Fig. 6B illustrates a calculated relationship between the optical-axis vector $V_{opt}\rightarrow$ and the visual-axis vector $V_{vis}\rightarrow$ replacing the relationship illustrated in Fig. 6A, the calculated relationship being obtained by performing the line-of-sight calibration.

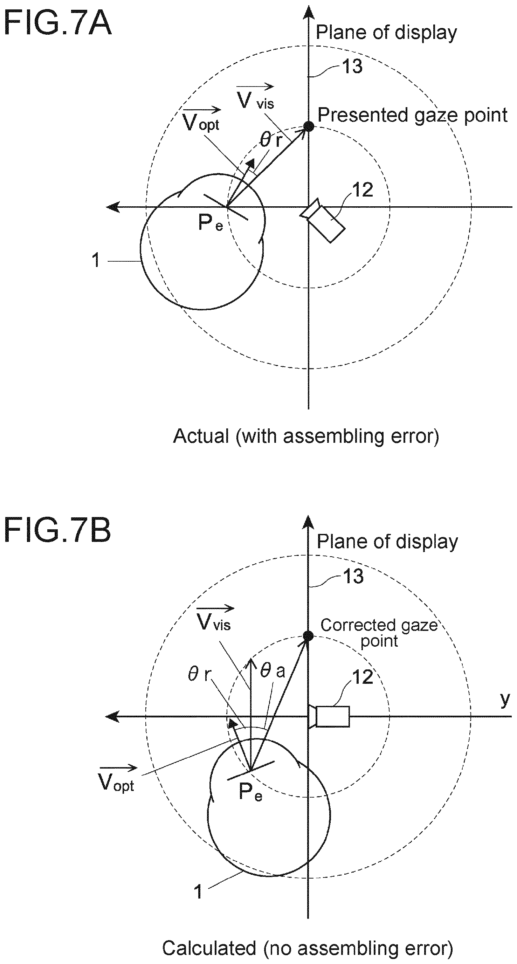

[Fig. 7A] Fig. 7A illustrates an actual relationship between an optical-axis vector $V_{opt}\rightarrow$ and a visual-axis vector $V_{vis}\rightarrow$ when there is an assembling error with respect to a pose of the image-capturing section 12 in the typical line-of-sight detection device.

[Fig. 7B] Fig. 7B illustrates a calculated relationship between the optical-axis vector $V_{opt}\rightarrow$ and the visual-axis vector $V_{vis}\rightarrow$ replacing the relationship illustrated in Fig. 7A, the calculated relationship being obtained by performing a line-of-sight calibration.

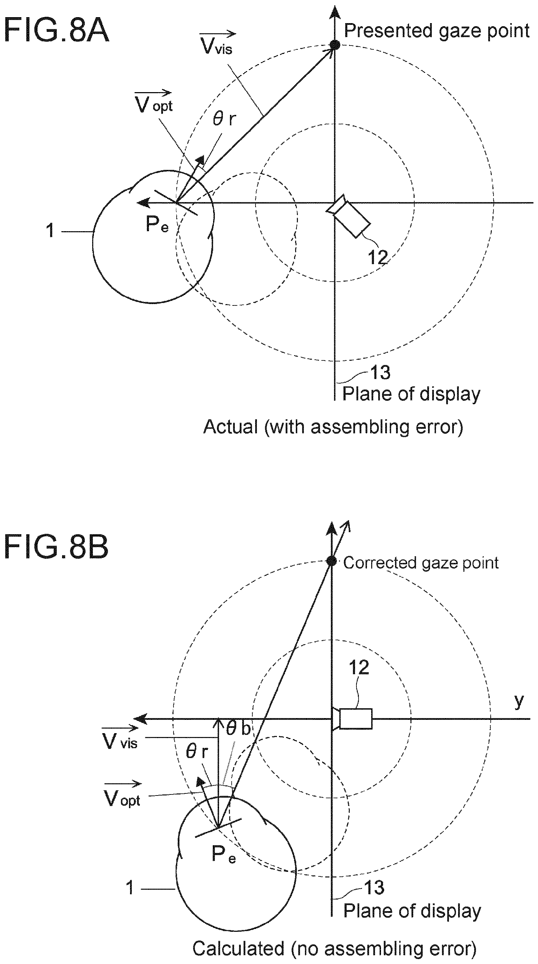

[Fig. 8A] Fig. 8A illustrates a relationship between an optical-axis vector $V_{opt}\rightarrow$ and a visual-axis vector $V_{vis}\rightarrow$ when the position of the line-of-sight-estimation-target eyeball 1 of the user moves in a direction of a radius of a position of the image-capturing section 12 with respect to the position upon performing a line-of-sight calibration.

[Fig. 8B] Fig. 8B illustrates a calculated relationship between the optical-axis vector $V_{opt}\rightarrow$ and the visual-axis vector $V_{vis}\rightarrow$ replacing the relationship illustrated in Fig. 8A, the calculated relationship being obtained by performing the line-of-sight calibration.

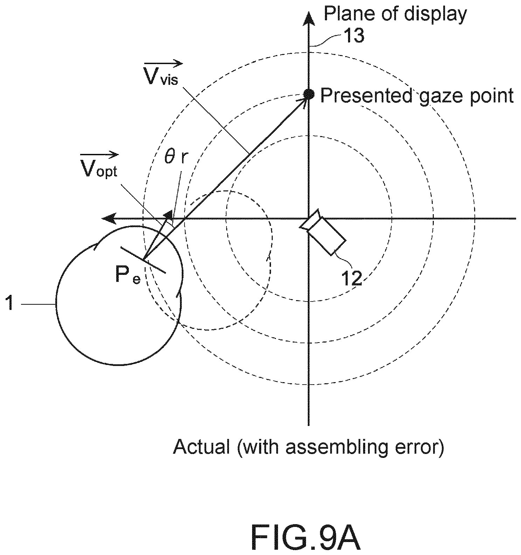

[Fig. 9A] Fig. 9A illustrates a relationship between an optical-axis vector $V_{opt}\rightarrow$ and a visual-axis vector $V_{vis}\rightarrow$ when the position of the line-of-sight-estimation-target eyeball 1 of the user moves in a direction other than the direction of the radius of the position of the image-capturing section 12 with respect to the position upon performing a line-of-sight calibration.

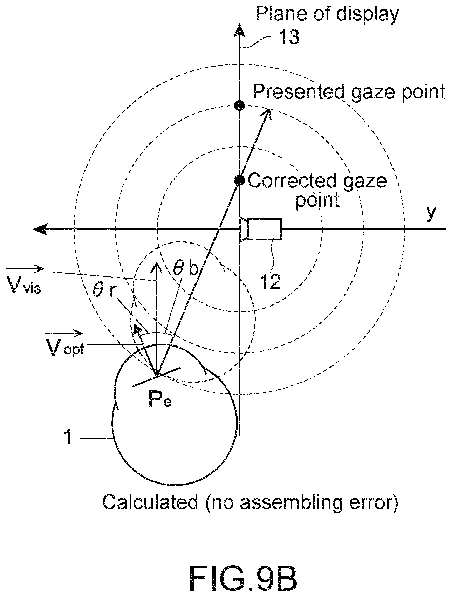

[Fig. 9B] Fig. 9B illustrates a calculated relationship between the optical-axis vector $V_{opt}\rightarrow$ and the visual-axis vector $V_{vis}\rightarrow$ replacing the relationship illustrated in Fig. 9A, the calculated relationship being obtained by performing the line-of-sight calibration.

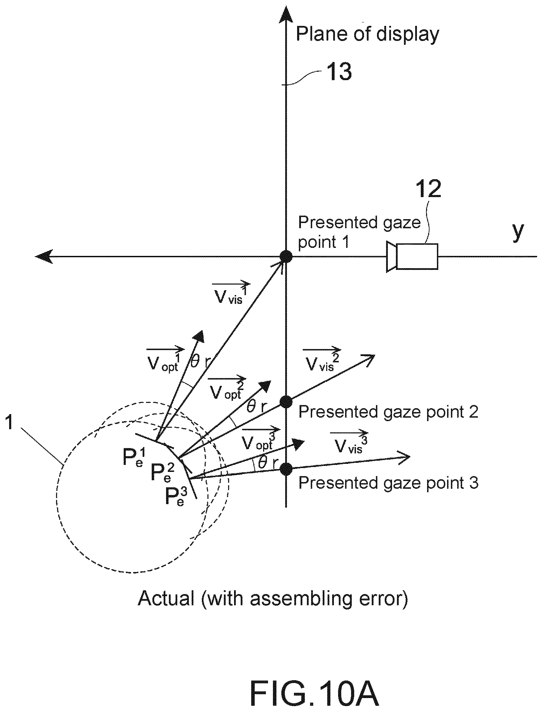

[Fig. 10A] Fig. 10A illustrates actual relationships between an optical-axis vector $V_{opt}^{n}\rightarrow$ and a visual-axis vector $V_{vis}^{n}\rightarrow$ with respect to N respective presented gaze points.

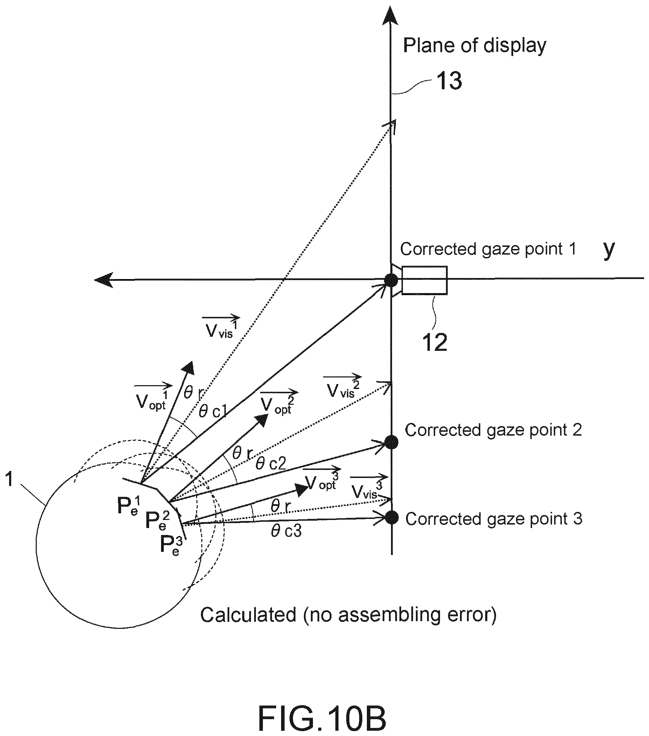

[Fig. 10B] Fig. 10B illustrates calculated relationships between the optical-axis vector $V_{opt}^{n}\rightarrow$ and the visual-axis vector $V_{vis}^{n}\rightarrow$ replacing the relationships illustrated in Fig. 10A, the calculated relationships being obtained by performing a line-of-sight calibration.

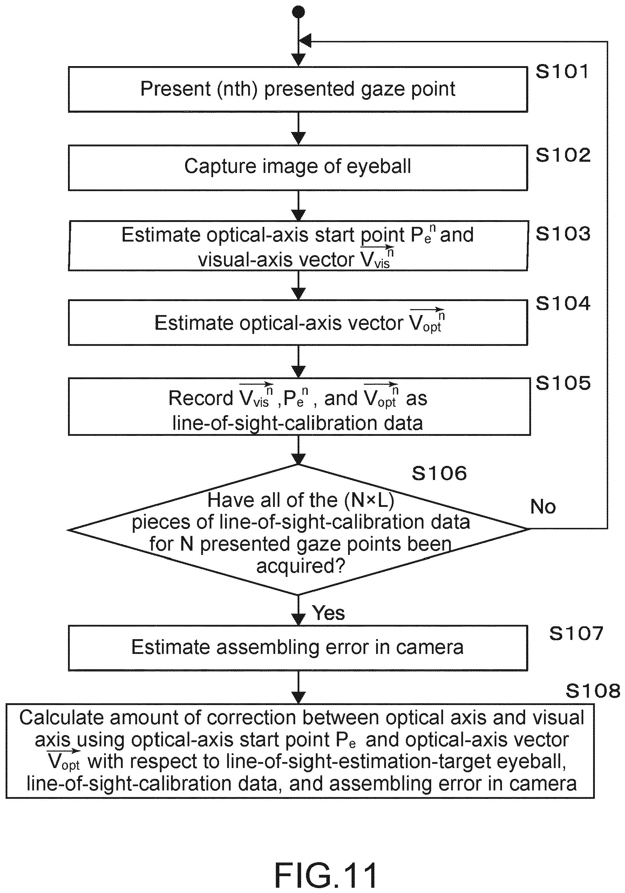

[Fig. 11] Fig. 11 is a flowchart illustrating a procedure of a method for calculating an amount of correction between an optical axis and a visual axis according to the first embodiment of the present technology, in which an assembling error in hardware is taken into consideration.

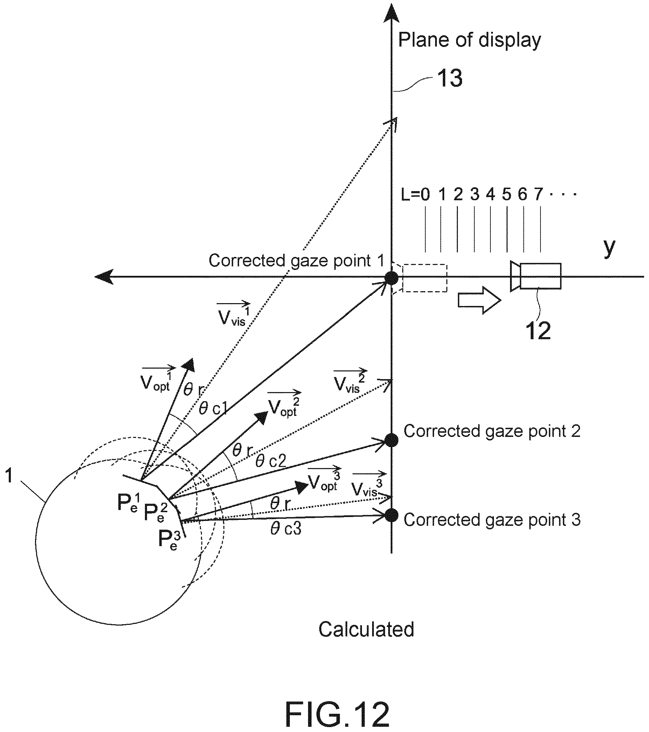

[Fig. 12] Fig. 12 is a diagram explaining a movement of the image-capturing section 12 when the method, of Fig. 11, for calculating an amount of correction between an optical axis and a visual axis in which an assembling error in hardware is taken into consideration.

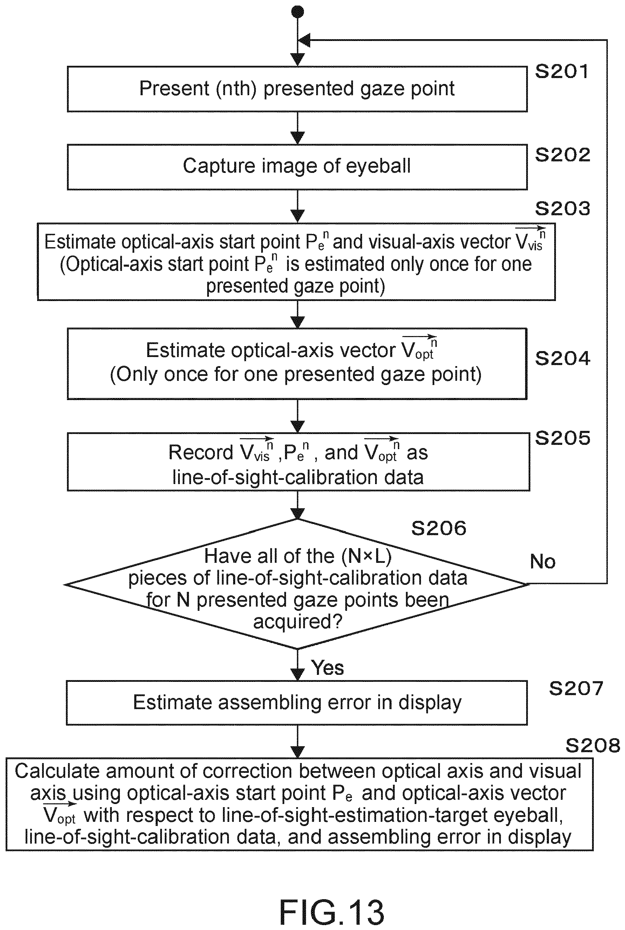

[Fig. 13] Fig. 13 is a flowchart illustrating a procedure of a method for calculating an amount of correction between an optical axis and a visual axis in which an assembling error in a display section 13 is taken into consideration.

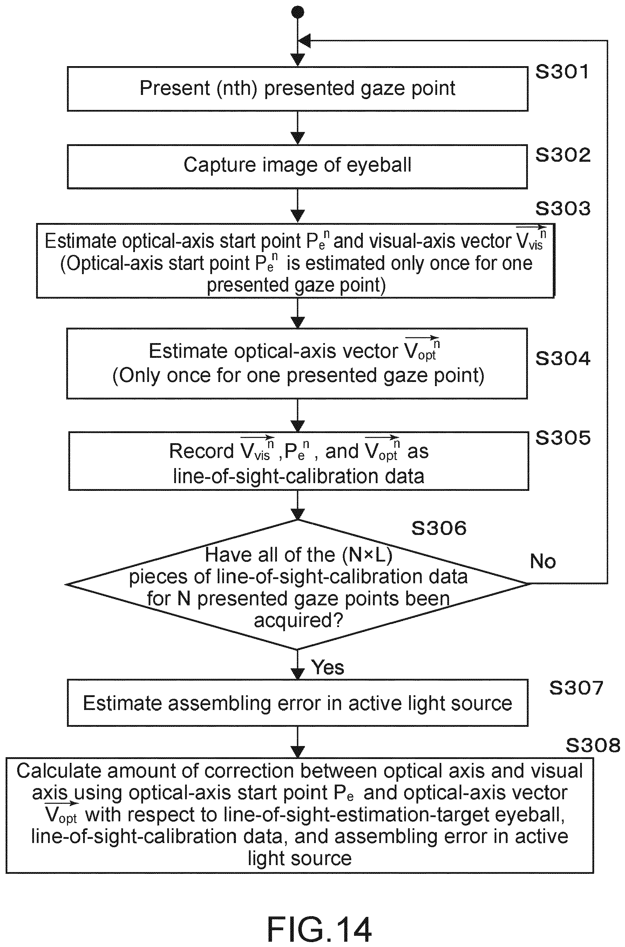

[Fig. 14] Fig. 14 is a flowchart illustrating a procedure of a method for calculating an amount of correction between an optical axis and a visual axis in which an assembling error in an active light source 11 is taken into consideration.

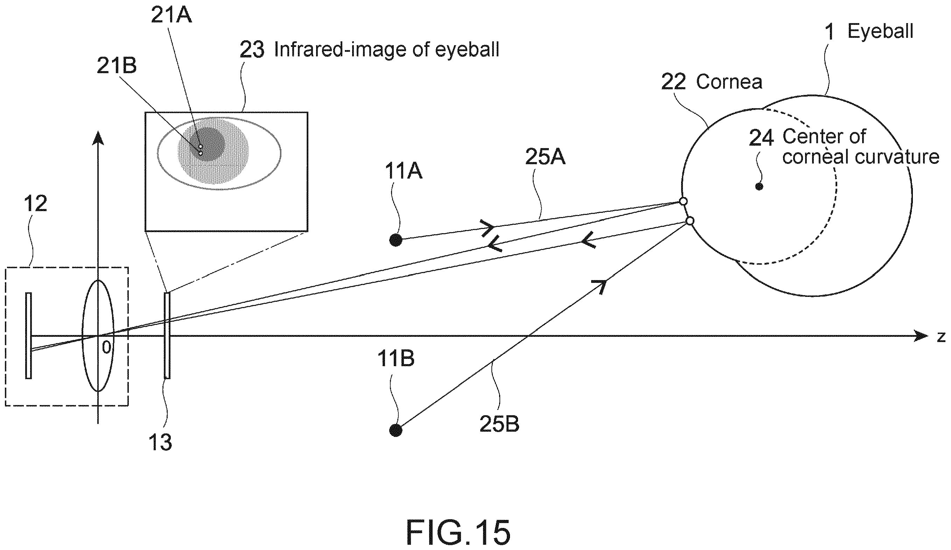

[Fig. 15] Fig. 15 illustrates an outline of a method for estimating a three-dimensional position of a center of a corneal curvature in a sensor-origin coordinate system.

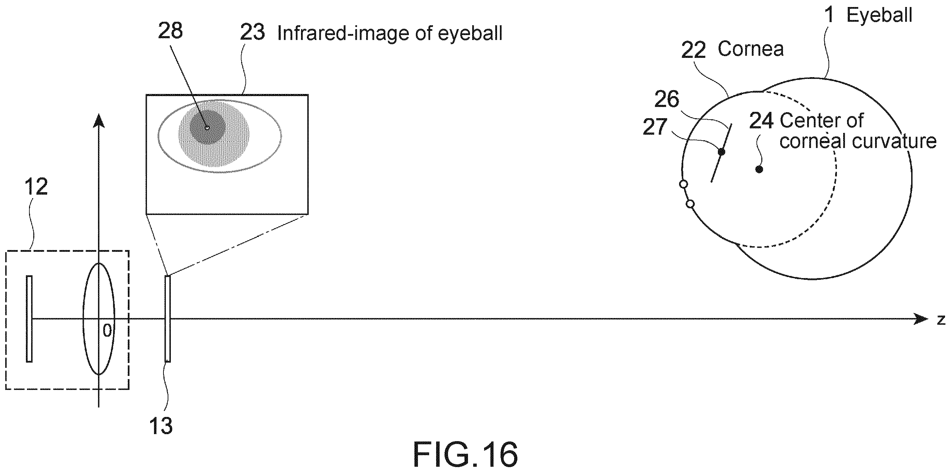

[Fig. 16] Fig. 16 illustrates an outline of a method for estimating a three-dimensional position of a center of a pupil in the sensor-origin coordinate system.

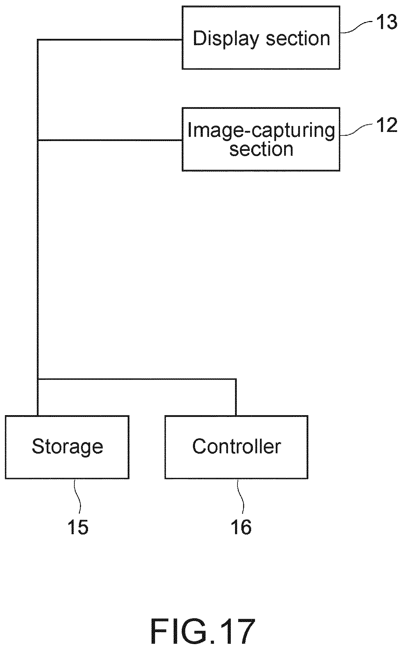

[Fig. 17] Fig. 17 is a block diagram of hardware configuration of a line-of-sight detection device that does not include the active light source.

Mode(s) for Carrying Out the Invention

Embodiments according to the present technology will now be described below with reference to the drawings.

<First Embodiment>

[Hardware Configuration of Information Processing Device]

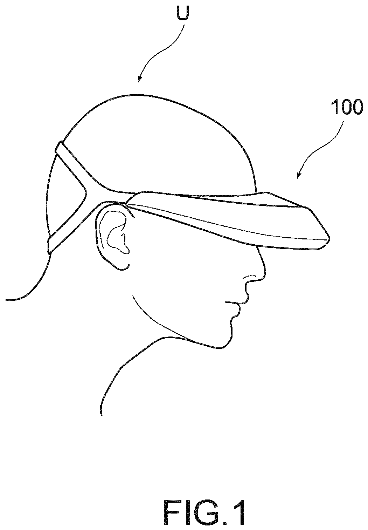

Fig. 1 illustrates a configuration of an information processing device 100 according to a first embodiment of the present technology.

The information processing device 100 is a device wearable on the head of a user U to cover the eyes of the user U, and is a so-called head-mounted display. The information processing device 100 includes a display section. A video such as a virtual reality (VR) video or an augmented reality (AR) video is displayed on a screen of the display section.

The information processing device 100 is provided with a line-of-sight detection device that detects where in a screen space of the display section the line of sight of the user U exists. In the information processing device 100, for example, a control of a display video is performed using data regarding a line of sight of the user U that is detected by the line-of-sight detection device.

[Line-of-Sight Detection Device]

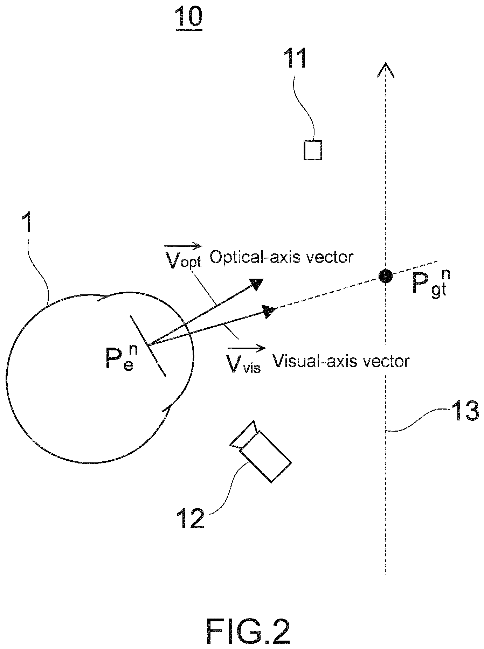

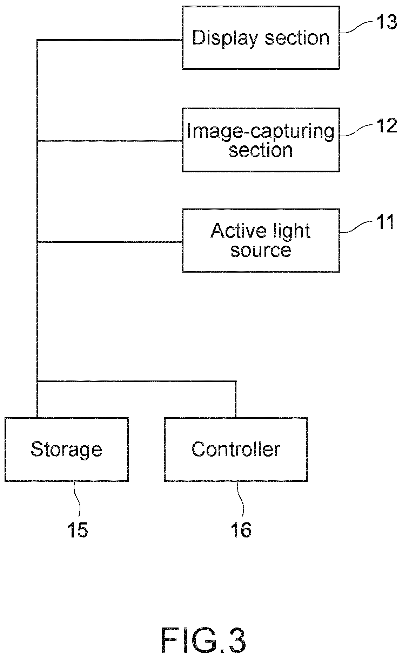

Next, a configuration of the line-of-sight detection device is described using Figs. 2 and 3.

Fig. 2 conceptually illustrates a configuration of a line-of-sight detection device 10.

Fig. 3 is a block diagram of a hardware configuration of the line-of-sight detection device 10. The line-of-sight detection device 10 includes an active light source 11, an image-capturing section 12, a display section 13, a storage 15, and a controller 16.

The active light source 11 is a light source that irradiates light for a line-of-sight calibration and a line-of-sight estimation onto an eyeball 1 of a user who is looking at an arbitrary position $P_{gt}{}^{n}$ on a screen of the display section 13. For example, an infrared light emitting diode (LED) is used as the active light source 11.

The image-capturing section 12 is, for example, a camera, and is a device that captures an image of light reflected from the eyeball 1 of the user, the eyeball 1 being irradiated with light from the active light source 11. The image-capturing section 12 includes, for example, a lens, an imaging element, and a signal processing circuit. Examples of the lens include an imaging lens that forms an optical image of a subject on an image-capturing surface of the imaging element. The imaging element includes, for example, an image sensor such as a complementary-metal-oxide-semiconductor (CMOS) image sensor and a charge-coupled-device (CCD) image sensor. The signal processing circuit includes, for example, an automatic-gain-control (AGC) circuit and an analog-to-digital converter (ADC), and convers an analog signal generated by the imaging element into a digital signal (image data). Further, the signal processing circuit performs various processes related to, for example, ROW image-development. Moreover, the signal processing circuit may perform various signal processes such as a white balance process, a color-tone correction process, a gamma process, a YCbCr conversion process, and an edge enhancement process.

The display section 13 is, for example, a liquid crystal display or an organic electro-luminescence (EL) display (also referred to as an organic light emitting diode (OLED) display). When a line-of-sight calibration is performed that estimates a difference between an optical-axis vector and a visual-axis vector of a user, a presented gaze point is displayed on the screen of the display section 13 by a control being performed by the controller 16, the presented gaze point being a point to be gazed at by the user.

The storage 15 includes a volatile memory and a nonvolatile memory. The volatile memory is, for example, a random access memory (RAM), and stores therein, for example, control data, such as a program and an operational parameter, that is used by the controller 16, and data of an image captured by the image-capturing section 12.

The nonvolatile memory stores therein, for example, various measurement data related to the eyeball 1 for each user and data used to perform measurement. For example, a flash memory is used as the nonvolatile memory. The nonvolatile memory may be removable from the body of the information processing device 100.

The controller 16 includes, for example, a central processing unit (CPU). The controller 16 performs arithmetic processing to control the information processing device 100 including the line-of-sight detection device 10, using the control data stored in the volatile memory and the various data stored in the nonvolatile memory.

[Line-of-Sight Estimation Method]

Next, a line-of-sight estimation method performed by the line-of-sight detection device 10 is described.

As the line-of-sight estimation method, there exists, for example, a method for estimating a line of sight using a three-dimensional model of an eyeball. Typically, first, the controller 16 estimates an optical-axis vector of an eyeball using an image (second image) of the eyeball that is captured by the image-capturing section 12. As a method for estimating an optical-axis vector, there exists, for example, a corneal reflection method that uses a corneal light reflex obtained by light from the active light source 11 being reflected off a cornea. In this method, first, a pupil is detected from a captured image. Next, the controller 16 sets, according to a position of the pupil that is specified from the captured image, a range in which a corneal light reflex exists, and estimates a three-dimensional position of a center of a corneal curvature and a three-dimensional position of a center of a pupil, using a position of observing the corneal light reflex and the pupil in the image. The controller 16 obtains, as a result of estimating an optical-axis vector, a vector that connects the estimated three-dimensional position of the center of a corneal curvature with the estimated three-dimensional position of the center of a pupil, and is oriented outward from the eyeball.

On the other hand, an orientation vector indicating a direction in which a man subjectively feels that he or she is looking, is called a visual-axis vector. The visual-axis vector is a vector that connects a fovea of an eyeball with a center of a cornea of the eyeball, and thus there exists a deviation (difference) of a visual-axis vector from an optical-axis vector. Thus, a visual-axis vector is estimated by correcting an optical-axis vector estimated as described above using a difference (an amount of correction) between an optical-axis vector and a visual-axis vector that is obtained by performing a line-of-sight calibration described later. The difference used to estimate a visual-axis vector from an optical-axis vector may be hereinafter referred to as an "amount of correction between an optical axis and a visual axis (a correction amount)" as appropriate.

[Line-of-Sight Calibration]

An outline of a typical line-of-sight calibration is described using Fig. 2.

In a typical line-of-sight calibration, a presented gaze point $P_{gt}^n$ is presented on the screen of the display section 13 to be gazed at by a user. Here, the controller 16 calculates a difference between an optical-axis vector $V_{opt}\rightarrow$ and a visual-axis vector $V_{vis}\rightarrow$, the optical-axis vector $V_{opt}\rightarrow$ connecting a three-dimensional position of a center $P_e^n$ of a corneal curvature with a three-dimensional position of a center of a pupil, the three-dimensional position of the center $P_e^n$ of a corneal curvature and the three-dimensional position of the center of a pupil being estimated using an image captured by the image-capturing section 12, the visual-axis vector $V_{vis}\rightarrow$ connecting the three-dimensional position $P_e^n$ of the center of a corneal curvature with a three-dimensional position $P_{gt}^n$ of the presented gaze point, the three-dimensional position $P_e^n$ of the center of a corneal curvature being estimated using the image captured by the image-capturing section 12.

However, due to an error (an assembling error) in an assembling state such as a position and a pose of a device such as the image-capturing section 12, the display section 13, and the active light source 11, there may occur a decrease in the accuracy in estimating, using a line-of-sight calibration, a difference between an optical-axis vector $V_{opt}\rightarrow$ and a visual-axis vector $V_{vis}\rightarrow$, that is, an amount of correction between an optical axis and a visual axis.

[Effects That Assembling Error Has on Line-of-Sight Calibration]

Effects that an assembling error of a device, and particularly, an assembling error of the image-capturing section 12, has on a line-of-sight calibration, are discussed below.

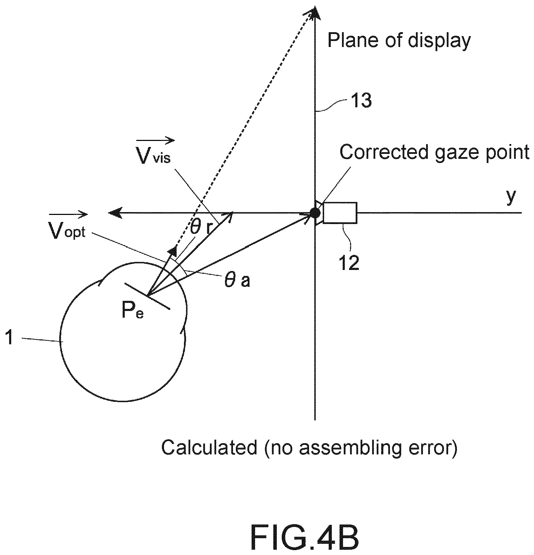

Figs. 4A and 4B each illustrate a relationship between an optical-axis vector $V_{opt}\rightarrow$ and a visual-axis vector $V_{vis}\rightarrow$ with respect to a presented gaze point when the image-capturing section 12 is arranged offset in an optical-axis direction y of the image-capturing section 12. Here, Fig. 4A illustrates an actual relationship between an optical-axis vector $V_{opt}\rightarrow$ and a visual-axis vector $V_{vis}\rightarrow$. Fig. 4B illustrates a calculated relationship between the optical-axis vector $V_{opt}\rightarrow$ and the visual-axis vector $V_{vis}\rightarrow$ replacing the relationship illustrated in Fig. 4A, the calculated relationship being obtained by performing a line-of-sight calibration (in the case of there being no assembling error).

In the presence of such an assembling error, when a line-of-sight calibration is performed while a user is gazing at a presented gaze point displayed at an intersection of the screen of the display section 13 and an optical axis y of the image-capturing section 12, a sum of an actual difference θr between an optical-axis vector $V_{opt}\rightarrow$ and a visual-axis vector $V_{vis}\rightarrow$, and a difference θa due to the assembling error with respect to the y-axis direction of the image-capturing section 12, is calculated as a calculated difference obtained by performing the line-of-sight calibration, as illustrated in Fig. 4B.

Regarding this difference (θr+θa), as illustrated in Figs. 5A and 5B, when the position of a line-of-sight-estimation-target eyeball 1 of a user moves in a direction orthogonal to the optical-axis direction y of the image-capturing section 12 with respect to a position upon performing a line-of-sight calibration (indicated by a dotted line), this results in obtaining a correct result of estimating a line of sight, in which there is an assembling error in the image-capturing section 12 with respect to the optical-axis direction y. Thus, there occurs no problem. However, as illustrated in Figs. 6A and 6B, when the position of the line-of-sight-estimation-target eyeball 1 of the user moves in a direction (for example, the optical-axis direction y) other than the direction orthogonal to the optical-axis direction y of the image-capturing section 12 with respect to the position upon performing a line-of-sight calibration, this results in a mismatch between a corrected gaze point and a presented gaze point, that is, this results in not obtaining a correct result of estimating a line of sight.

Next, the case of there being an assembling error with respect to a pose of the image-capturing section 12, is discussed.

Figs. 7A and Fig. 7B each illustrate a relationship between an optical-axis vector $V_{opt}\rightarrow$ and a visual-axis vector $V_{vis}\rightarrow$ with respect to a presented gaze point when there is an assembling error with respect to a pose of the image-capturing section 12. Here, Fig. 7A illustrates an actual relationship between an optical-axis vector $V_{opt}\rightarrow$ and a visual-axis vector $V_{vis}\rightarrow$. Fig. 7B illustrates a calculated relationship between the optical-axis vector $V_{opt}\rightarrow$ and the visual-axis vector $V_{vis}\rightarrow$ replacing the relationship illustrated in Fig. 7A, the calculated relationship being obtained by performing a line-of-sight calibration (in the case of there being no assembling error).

When a line-of-sight calibration is performed in the presence of an assembling error with respect to the pose of the image-capturing section 12, as described above, a sum of an actual difference $\theta r$ between an optical-axis vector $V_{opt}\rightarrow$ and a visual-axis vector $V_{vis}\rightarrow$, and a difference $\theta b$ due to an assembling error with respect to the pose of the image-capturing section 12, is calculated as a calculated difference obtained by performing the line-of-sight calibration, as illustrated in Fig. 7B.

Regarding this difference ($\theta r+\theta b$), as illustrated in Figs. 8A and 8B, when the position of an eyeball 1 moves in a direction of a radius of a position of the image-capturing section 12 with respect to a position upon performing a line-of-sight calibration (indicated by a dotted line), this results in obtaining a correct result of estimating a line of sight. Thus, there occurs no problem. However, as illustrated in Figs. 9A and 9B, when the position of the eyeball 1 moves in a direction other than the direction of the radius of the position of the image-capturing section 12 with respect to the position upon performing a line-of-sight calibration, this results in a mismatch between a corrected gaze point and a presented gaze point, that is, this results in not obtaining a correct result of estimating a line of sight.

As described above, when there is an assembling error in the image-capturing section 12, a line of sight is not correctly estimated except for a specific state. Likewise, when there is an assembling error in other hardware such as the display section 13 or the active light source 11, a line of sight is not correctly estimated except for a specific state.

[Line-of-Sight Calibration with Respect to N Presented Gaze Points]

Next, a typical method for estimating an amount of correction between an optical axis and a visual axis by performing a line-of-sight calibration with respect to N (a plurality of) presented gaze points, is described.

Figs. 10A and Fig. 10B each illustrate relationships between an optical-axis vector $V_{opt}^{n}\rightarrow$ and a visual-axis vector $V_{vis}^{n}\rightarrow$ with respect to N respective presented gaze points. Fig. 10A illustrates actual relationships between an optical-axis vector $V_{opt}^{n}\rightarrow$ and a visual-axis vector $V_{vis}^{n}\rightarrow$ with respect to N (three in this example) respective presented gaze points. Fig. 10B illustrates calculated relationships between the optical-axis vector $V_{opt}^{n}\rightarrow$ and the visual-axis vector $V_{vis}^{n}\rightarrow$ with respect to the N respective presented gaze points, the calculated relationships replacing the relationships illustrated in Fig. 10A, the calculated relationships being obtained by performing a line-of-sight calibration (in the case of there being no assembling error).

Anatomically, a geometric relationship between an optical axis and a visual axis of a user remains unchanged regardless of the direction of his or her line of sight. However, for example, when there is an assembling error in hardware such as the image-capturing section 12, a variation in an amount of correction between an optical axis and a visual axis occurs depending on the direction of a line of sight. In other words, an amount of correction between an optical axis and a visual axis with respect to a presented gaze point 1 is a sum of an actual difference $\theta r$ between an optical-axis vector $V_{opt}^{1}\rightarrow$ and a visual-axis vector $V_{vis}^{1}\rightarrow$, and a difference $\theta c1$ due to an assembling error in the image-capturing section 12. An amount of correction between an optical axis and a visual axis with respect to a presented gaze point 2 is a sum of an actual difference $\theta r$ between an optical-axis vector $V_{opt}^{2}\rightarrow$ and a visual-axis vector $V_{vis}^{2}\rightarrow$, and a difference $\theta c2$ due to the assembling error in the image-capturing section 12. An amount of correction between an optical axis and a visual axis with respect to a presented gaze point 3 is a sum of an actual difference $\theta r$ between an optical-axis vector $V_{opt}^{3}\rightarrow$ and a visual-axis vector $V_{vis}^{3}\rightarrow$, and a difference $\theta c3$ due to the assembling error in the image-capturing section 12. Since the three differences described above $\theta c1$, $\theta c2$, and $\theta c3$ due to the assembling error in the image-capturing section 12, are different from one another depending on the direction of a line of sight, a variation in an amount of correction between an optical axis and a visual axis occurs depending on the direction of a line of sight.

As described above, regarding amounts of correction between an optical axis and a visual axis that are respectively obtained by performing a line-of-sight calibration with respect to N respective presented gaze points, a variation occurs depending on the direction of a line of sight due to an assembling error in hardware. Thus, typically, for example, a method has been discussed that includes estimating, using an image, an optical-axis start point and an optical-axis vector of a line-of-sight-estimation-target eyeball of a user; and calculating an amount of correction between an optical axis and a visual axis using a similarity between the estimated optical-axis start point and an optical-axis start point saved as line-of-sight-calibration data, and a similarity between the estimated optical-axis vector and an optical-axis vector saved as the line-of-sight-calibration data, and using a correction amount for each presented gaze point included in the line-of-sight-calibration data. However, this is not a method in which the precision in hardware assembly is not explicitly considered, so it is still difficult to efficiently suppress the occurrence of an error in an amount of correction between an optical axis and a visual axis due to an assembling error in hardware.

Thus, in the information processing device 100 according to the first embodiment of the present technology, the controller 16 changes the position of a presented gaze point to display the presented gaze point at a plurality of different positions on the display section 13, according to control data such as a program stored in a RAM 14. Then, using the image-capturing section 12, the controller 16 captures, for each displayed presented gaze point, an image of an eyeball of a user who is gazing at the displayed presented gaze point, and generates a plurality of pieces of line-of-sight-calibration data corresponding to each of the presented gaze points. Then, using the plurality of pieces of line-of-sight-calibration data, the controller 16 estimates a position and a pose of an arbitrary piece of hardware included in the information processing device 100 when a variance of (or a difference between) differences between an optical-axis vector and a visual-axis vector for each of the presented gaze points, is minimum in the line-of-sight-calibration data. The estimated position and pose of the arbitrary piece of hardware can be considered a position and a pose in which an assembling error in the piece of hardware has been reflected. In other words, an effect of an assembling error in an arbitrary piece of hardware is reduced by calculating, using an estimated position and an estimated pose of the arbitrary piece of hardware, an amount of correction between an optical axis and a visual axis in which the assembling error in the arbitrary piece of hardware has been reflected. Note that, for example, one of the image-capturing section 12, the display section 13, and the light source 11, or a combination of two or more thereof may be selected as an arbitrary piece of hardware. This is described in detail below.

Note that, with respect to a camera, a display, and a light source that irradiates an eyeball of a user with light, values of design information acquired in advance are used for a position and a pose of each of the pieces of hardware, but a difference from the design information is estimated by the controller 16 to obtain a position and a pose after an assembling error occurs, since the difference from the design information is actually caused due to an effect of the assembling error. An original position in this case is stated in the design information.

[Calculation of Amount of Correction Between Optical Axis and Visual Axis in Which Assembling Error in Hardware is Taken into Consideration]

Fig. 11 is a flowchart illustrating a procedure of a method for calculating an amount of correction between an optical axis and a visual axis according to the first embodiment, in which an assembling error in hardware is taken into consideration.

Note that it is assumed that there is an assembling error in the image-capturing section 12 that is the assembling error in hardware.

1. First, the controller 16 controls the display section 13 such that a firstly presented gaze point (a first presented gaze point) from among N presented gaze points is displayed at a specified position (a first position) on the screen of the display section 13 (Step S101).

Here, the presented gaze point is a point (a virtual object) at which a user has been instructed to gaze, and is, for example, a round shape displayed on the display section 13.

2. Next, using the image-capturing section 12, the controller 16 repeatedly captures, L times, an image of the eyeball 1 of a user who is gazing at the firstly presented gaze position, while changing at least one of a position or a pose of the image-capturing section 12. Accordingly, the controller acquires L images (first images) of the eyeball that correspond to the firstly presented gaze position (Step S102). The value of L is limited due to a speed of the information processing device 1, but is favorably the largest possible number for a reason of accuracy.

Note that, as a method for changing at least one of a position or a pose of the image-capturing section 12, there exist a method for virtually changing at least one of the position or the pose of the image-capturing section 12 in a virtual space, and a method for actually changing at least one of the position or the pose of the image-capturing section 12 in a real space. When virtually changing at least one of the position or the pose of the image-capturing section 12, the controller 12 changes a calculated position or a calculated pose of the image-capturing section 12 in the virtual space. On the other hand, when actually changing at least one of the position or the pose of the image-capturing section 12, an adjustment section is added to the head-mounted display.

The adjustment section is capable of adjusting at least one of a position or a pose of the image-capturing section 12. Note that the adjustment section 12 may be capable of adjusting at least one of a position or a pose of the display section 13 and adjusting at least one of a position or a pose of the active light source 11, in addition to adjusting at least one of the position or the pose of the image-capturing section 12 (the cases of adjusting the position and the pose of the display section 13 and adjusting the position and the pose the active light source 11, which will be described later).

The adjustment section may be capable of automatically performing the adjustment described above using a drive section such as an actuator. Alternatively, the adjustment section may be capable of manually performing the adjustment described above (for example, a dialing mechanism).

When the adjustment described above is manually performed, the controller 12 controls the display section to display, to a user, guidance indicating how to move the controller 12 and a speed for moving the controller 12. For example, guidance, such as "Move more slowly", "Move more broadly", "Move similarly to the first movement", and "Move again", is displayed to be reported to a user. Note that audio guidance may be reported to a user.

The controller 12 may determine which of the various pieces of guidance described above is to be presented to a user, according to a condition such as whether a specified number (L) of pieces of line-of-sight-calibration data have been acquired with respect to the movement of the camera 12 in a certain range. Alternatively, the controller 12 may determine which of the various pieces of guidance described above is to be presented to a user, according to a condition such as whether the camera 12 has been moved on the same route for each presented gaze point.

Using the image-capturing section 12, the controller 16 captures an image of the eyeball 1 of the user (after guidance is displayed) while changing at least one of a position or a pose of the image-capturing section 12, and acquires position-and-pose information related to a position and a pose of the image-capturing section 12 in a virtual space or in a real space. The position-and-pose information may be considered information depending on a change in at least one of the position or the pose of the image-capturing section 13, the information including first position-and-pose information and second position-and-pose information that are different from each other. Then, the controller 16 associates the position-and-pose information regarding a position and a pose of the image-capturing section 12 with an image (first image) of the eyeball that is captured in a state in which the image-capturing section 12 is in the pose in the position, and saves them in the storage 14.

3. With respect to each of the L captured images (first images), the controller 16 estimates a three-dimensional position $P_e^n$ of a center of a corneal curvature, and estimates a visual-axis vector $V_{vis}^n\rightarrow$ that connects the three-dimensional position $P_e^n$ of the center of a corneal curvature with a three-dimensional position $P_{gt}^n$ of a presented gaze point (Step S103). Here, n represents numbers from 1 to N assigned to N respective presented gaze points.

4. With respect to each of the L captured images, the controller 16 estimates a three-dimensional position of a center of a pupil, and estimates an optical-axis vector $V_{opt}^n\rightarrow$ that connects the three-dimensional position of the center of a pupil with the three-dimensional position $P_e^n$ of the center of a corneal curvature (Step S104).

5. The controller 16 saves, in the storage 14 and as line-of-sight-calibration data, the estimated visual-axis vector $V_{vis}^n\rightarrow$, the estimated optical-axis vector $V_{opt}^n\rightarrow$, and the estimated three-dimensional position $P_e^n$ of the center of a corneal curvature described above (Step S105). Note that the controller 16 associates L pieces of calibration data with respective pieces of position-and-pose information, and stores them in the storage. The three-dimensional position $P_e^n$ of the center of a corneal curvature may also be hereinafter referred to as a position of an optical-axis start point.

As a result of performing the processes of Step S102 to Step S105 described above, the controller 16 acquires L pieces of line-of-sight-calibration data for one presented gaze point.

6. Next, the controller 16 determines whether all of the (N×L) pieces of line-of-sight-calibration data for the N presented gaze points have been acquired (Step S106). When a piece of line-of-sight-calibration data to be acquired remains (NO in Step S106), the controller 16 returns to the process of Step S101. Then, for example, the controller 16 moves the firstly presented gaze point to a specified position (a second position) on the screen of the display section 13, and controls the display section 13 to display a secondly presented gaze point (a second presented gaze point).

When the secondly presented gaze point or a presented gaze point subsequent to the secondly presented gaze point is presented, the controller 16 changes the position and the pose of the image-capturing section 12 (in a virtual space or in a real space) by a change method similar to the method performed when the firstly presented gaze point is presented.

7. When all of the (L×N) pieces of line-of-sight-calibration data have been acquired (YES in Step S106), the controller 16 estimates an assembling error in the image-capturing section 12 as indicated below (Step S107).

The controller 16 calculates differences between an optical-axis vector $V_{opt}^n\rightarrow$ and a visual-axis vector $V_{vis}^n\rightarrow$ with respect to the (L×N) respective acquired pieces of line-of-sight-calibration data. Using the pieces of position-and-pose information (pieces of first position-and-pose information and pieces of second position-and-pose information) associated with the respective pieces of line-of-sight-calibration data, the controller 16 classifies, by the same position and the same pose of the image-capturing section 12, the differences between an optical-axis vector $V_{opt}^n\rightarrow$ and a visual-axis vector $V_{vis}^n\rightarrow$ that are calculated with respect to the (L×N) respective pieces of line-of-sight-calibration data.

Consequently, the (L×N) pieces of line-of-sight-calibration data are classified into L groups each including N pieces of line-of-sight-calibration data. The N pieces of line-of-sight-calibration data (pieces of first calibration data: pieces of calibration data in a first group, and pieces of second calibration data: pieces of calibration data in a second group) in each of the L groups created by the classification, are pieces of data acquired in a state in which the image-capturing section 12 is in the same pose in the same position (in a virtual space or in a real space). Further, as described above, the N pieces of line-of-sight-calibration data in each of the L groups created by the classification each include information such as a visual-axis vector $V_{vis}^n\rightarrow$, an optical-axis vector $V_{opt}^n\rightarrow$, a three-dimensional position $P_e^n$ of a

center of a corneal curvature, and a difference between the optical-axis vector $V_{opt}^n \rightarrow$ and the visual-axis vector $V_{vis}^n \rightarrow$. Note that line-of-sight-calibration data may include information regarding a three-dimensional position of a center of an eyeball rotation.

The controller 16 determines a position and a pose having a minimum variance of differences between an optical-axis vector $V_{opt}^n \rightarrow$ and a visual-axis vector $V_{vis}^n \rightarrow$ for each classification group of pieces of data (for each of the L groups). Then, from the determined position and pose, the controller 16 calculates an error with respect to a design position and a design pose of the image-capturing section 12 as an assembling error.

Return to the description of an operation of calculating an amount of correction between an optical axis and a visual axis in which an assembling error in hardware is taken into consideration.

8. After the estimation of an assembling error in the image-capturing section 12 is completed, it becomes possible to perform an operation (a line-of-sight estimation) that includes calculating an amount of correction between an optical axis and a visual axis with respect to an optical-axis vector $V_{opt} \rightarrow$ of the eyeball 1 of the user, and estimating a visual-axis vector $V_{vis} \rightarrow$.

In the line-of-sight estimation, the controller 16 captures an image of the line-of-sight-estimation-target eyeball 1 of the user using the image-capturing section 12, and acquires an image (second image) of the eyeball of the user. The controller 16 estimates, from the captured image, a three-dimensional position $P_e$ of the center of a corneal curvature and a three-dimensional position of the center of a pupil, and estimates a vector connecting them as an optical-axis vector $V_{opt} \rightarrow$.

Here, an optical-axis vector $V_{opt} \rightarrow$ of the line-of-sight-estimation-target eyeball 1 of the user in which an assembling error in hardware is taken into consideration, is estimated by estimating the three-dimensional position of the center $P_e$ of a corneal curvature and the three-dimensional position of the center of a pupil while taking into consideration the assembling error in hardware. From the optical-axis vector $V_{opt} \rightarrow$ and the three-dimensional position $P_e$ of the center of a corneal curvature that are estimated in which the assembling error in hardware is taken into consideration, and from N pieces of line-of-sight-calibration data from among the $(L \times N)$ pieces of line-of-sight-calibration data, the N pieces of line-of-sight-calibration data being N pieces of line-of-sight-calibration data at least having a minimum variance of differences between an optical-axis vector $V_{opt}^n \rightarrow$ and a visual-axis vector $V_{vis}^n \rightarrow$, the controller 16 estimates, as an amount of correction between an optical axis and a visual axis, a difference between the optical-axis vector $V_{opt} \rightarrow$ and a visual-axis vector $V_{vis} \rightarrow$ of the line-of-sight-estimation-target eyeball 1 of the user in which the assembling error in hardware is taken into consideration. Then, the controller 16 corrects the optical-axis vector $V_{opt} \rightarrow$ using the amount of correction between an optical axis and a visual axis to estimate the visual-axis vector, and this results in performing a line-of-sight estimation.

Note that, in the present embodiment, an example of using, for correction, N pieces of line-of-sight-calibration data from among $(L \times N)$ pieces of line-of-sight-calibration data is described, the N pieces of line-of-sight-calibration data being N pieces of line-of-sight-calibration data having a minimum variance of differences between an optical-axis vector $V_{opt}^n \rightarrow$ and a visual-axis vector $V_{vis}^n \rightarrow$. Here, there is a possibility that N is two. The case in which N is two is the case in which the number of presented gaze points is two and the number of pieces of line-of-sight-calibration data in a group is two. In this case, for example, the controller 16 uses, for correction, N (N=2) pieces of line-of-sight-calibration data from among $(L \times N$ (N=2)) pieces of line-of-sight-calibration data, the N pieces of line-of-sight-calibration data being N pieces of line-of-sight-calibration data having a smaller difference between a certain difference between an optical-axis vector $V_{opt}^n \rightarrow$ and a visual-axis vector $V_{vis}^n \rightarrow$, and another difference between an optical-axis vector and a visual-axis vector, the certain difference corresponding to a firstly presented gaze point (a first presented gaze point), the other difference corresponding to a secondly presented gaze point (a second presented gaze point).

[Method for Calculating Amount of Correction Between Optical Axis and Visual Axis]

Next, an example of a method for calculating an amount of correction between an optical axis and a visual axis, is described.

The controller 16 calculates a similarity $D^n$ between a three-dimensional position $P_e$ of a center of a corneal curvature an optical-axis vector $V_{opt} \rightarrow$ of the line-of-sight-estimation-target eyeball 1 of the user, and a three-dimensional position $P_e^n$ of the center of a corneal curvature and an optical-axis vector $V_{opt}^n \rightarrow$ with respect to each of the N pieces of line-of-sight-calibration data, the three-dimensional position $P_e$ of the center of a corneal curvature and the optical-axis vector $V_{opt} \rightarrow$ being estimated, with an assembling error in hardware being taken into consideration. Here, the controller 16 may calculate the similarity $D^n$ using an angle $\varphi^n$ formed by $V_{opt} \rightarrow$ and $V_{opt}^n \rightarrow$ as an index. Regarding the other examples, the cosine similarity, the Euclidean distance between $P_e$ and $P_e^n$, or a combination of two or more thereof may be used as an index of the similarity $D^n$.

Next, the controller 16 obtains a similarity $//D^n//$ by normalizing $D^n$. For example, when the similarity is calculated using the angle $\varphi^n$ formed by $V_{opt} \rightarrow$ and $V_{opt}^n \rightarrow$ as an index, $//D^n//$ can be obtained using $1/\varphi^n$. Here, an exceptional process such as the following is performed when $\varphi^n=0$: $//D^n//=1$, and a normalized similarity for a point other than the nth point is set to "0".

Next, the controller 16 weights, using a normalized similarity as a coefficient, N differences between an optical-axis vector $V_{opt}^n \rightarrow$ and a visual-axis vector $V_{vis}^n \rightarrow$ with respect to N pieces of line-of-sight-calibration data, and calculates a value obtained by adding all of the results as an amount of correction between an optical axis and a visual axis with respect to an optical-axis vector $V_{opt} \rightarrow$ of the line-of-sight-estimation-target eyeball 1 of the user.

Note that the controller 16 may calculate an amount of correction between an optical axis and a visual axis as indicated below. First, using an image (a second image) of an eyeball upon performing a line-of-sight estimation, the controller 16 estimates a three-dimensional position of a center of a corneal curvature and a three-dimensional position of a center of a pupil or a center of an eyeball rotation. Then, the controller 16 determines a similarity between the estimated three-dimensional position of the center of a corneal curvature and the estimated three-dimensional position of the center of a pupil or the center of an eyeball rotation, and another three-dimensional position of the center of a corneal curvature and another three-dimensional position of the center of a pupil or the center of an eyeball rotation, the other three-dimensional position of the center of a corneal curvature and the other three-dimensional position of the center of a pupil or the center of an eyeball rotation being included in each of the N pieces of calibration data from among (L×N) pieces of line-of-sight-calibration data, the N pieces of calibration data being N pieces of calibration data in which the variance described above (or the difference described above) is minimum. Then, using the determined similarity, the controller 16 calculates an amount of correction between an optical axis and a visual axis. Note that the similarity in this case is evaluated in combination with the similarity of an optical-axis vector.

The method for estimating an assembling error and the line-of-sight estimation in which the estimated assembling error is taken into consideration, have been described above on the assumption that, in particular, the image-capturing section 12 is assembling-error-estimation-target hardware, but the method for estimating an assembling error and the line-of-sight estimation in which the estimated assembling error is taken into consideration, can also be similarly performed when the display section 13 or the active light source 11 is an assembling-error-estimation target.

[Method for Estimating Assembling Error in Display Section 13]

Next, a method for calculating an amount of correction between an optical axis and a visual axis in which an assembling error in hardware is taken into consideration, is described in detail, where the display section 13 is assembling-error-estimation-target hardware.

Fig. 13 is a flowchart illustrating a procedure of a method for calculating an amount of correction between an optical axis and a visual axis in which an assembling error in the display section 13 is taken into consideration.

Note that, in essence, a processing procedure in this case is similar to the method for calculating an amount of correction between an optical axis and a visual axis in which an assembling error in the image-capturing section 12 is taken into consideration.

11. First, the controller 16 controls the display section 13 such that a firstly presented gaze point from among N presented gaze points is displayed at a specified position on the screen of the display section (Step S201).

12. Next, using the image-capturing section 12, the controller 16 repeatedly captures, L times, an image of the eyeball 1 of a user who is gazing at the firstly presented gaze position, while changing at least one of a position or a pose of the display section 13. Accordingly, the controller 16 acquires L images (first images) of the eyeball that correspond to the firstly presented gaze position (Step S202). The value of L is limited due to a speed of the information processing device 1, but is favorably the largest possible number for a reason of accuracy.

Using the image-capturing section 12, the controller 16 captures an image of the eyeball 1 of the user while changing at least one of a position or a pose of the display section 13, and acquires position-and-pose information related to a position and a pose of the display section 13 in a virtual space or in a real space. The position-and-pose information may be considered information depending on a change in at least one of the position or the pose of the display section 13, the information including first position-and-pose information and second position-and-pose information that are different from each other. Then, the controller 16 associates the position-and-pose information regarding a position and a pose of the display section 13 with an image (first image) of the eyeball that is captured in a state in which the display section 13 is in the pose in the position, and saves them in the storage 14.

13. With respect to each of the L captured images, the controller 16 estimates a three-dimensional position $P_e^n$ of the center of a corneal curvature, and estimates a visual-axis vector $V_{vis}^n \rightarrow$ that connects the three-dimensional position $P_e^n$ of the center of a corneal curvature with a three-dimensional position $P_{gt}^n$ of a presented gaze point (Step S203). Note that, since the three-dimensional position $P_e^n$ of the center of a corneal curvature does not depend on a position or a pose of the display section 13, it is sufficient if the three-dimensional position $P_e^n$ of the center of a corneal curvature is estimated once for one presented gaze point.

14. With respect to each of the L captured images, the controller 16 estimates a three-dimensional position of a center of a pupil, and estimates an optical-axis vector $V_{opt}^n \rightarrow$ that connects the three-dimensional position of the center of a pupil with the three-dimensional position $P_e^n$ of the center of a corneal curvature (Step S204). Here, it is sufficient if the optical-axis vector $V_{opt}{}^n\rightarrow$ is estimated once for one presented gaze point.

15. The controller 16 saves, in the storage 14 and as line-of-sight-calibration data, the estimated visual-axis vector $V_{vis}{}^n\rightarrow$, the estimated optical-axis vector $V_{opt}{}^n\rightarrow$, and the estimated three-dimensional position $P_e{}^n$ of the center of a corneal curvature described above (Step S205).

As a result of performing the processes of Step S202 to Step S205 described above, the controller 16 acquires L pieces of line-of-sight-calibration data for one presented gaze point.

16. Next, the controller 16 determines whether all of the ($N\times L$) pieces of line-of-sight-calibration data for the N presented gaze points have been acquired (Step S206). When a piece of line-of-sight-calibration data to be acquired remains (NO in Step S206), the controller 16 returns to the process of Step S201. Then, for example, the controller 16 moves the firstly presented gaze point to a specified position (a second position) on the screen of the display section 13, and controls the display section 13 to display a secondly presented gaze point (a second presented gaze point).

When the secondly presented gaze point or a presented gaze point subsequent to the secondly presented gaze point is presented, the controller 16 changes the position and the pose of the display section 13 (in a virtual space or in a real space) by a change method similar to the method performed when the firstly presented gaze point is presented.

17. When all of the ($L\times N$) pieces of line-of-sight-calibration data have been acquired (YES in Step S206), the controller 16 estimates an assembling error in the display section 13 as indicated below (Step S207).

The controller 16 calculates differences between an optical-axis vector $V_{opt}{}^n\rightarrow$ and a visual-axis vector $V_{vis}{}^n\rightarrow$ with respect to the ($L\times N$) respective acquired pieces of line-of-sight-calibration data. Using the pieces of position-and-pose information (pieces of first position-and-pose information and pieces of second position-and-pose information) associated with the respective pieces of line-of-sight-calibration data, the controller 16 classifies, by the same position and the same pose of the display section 13, the differences between an optical-axis vector $V_{opt}{}^n\rightarrow$ and a visual-axis vector $V_{vis}{}^n\rightarrow$ that are calculated with respect to the ($L\times N$) respective pieces of line-of-sight-calibration data.

Consequently, the ($L\times N$) pieces of line-of-sight-calibration data are classified into L groups each including N pieces of line-of-sight-calibration data. The N pieces of line-of-sight-calibration data in each of the L groups created by the classification, are pieces of data acquired in a state in which the display section 13 is in the same pose in the same position (in a virtual space or in a real space).

The controller 16 determines a position and a pose of the display section 13 having a minimum variance of differences between an optical-axis vector $V_{opt}{}^n\rightarrow$ and a visual-axis vector $V_{vis}{}^n\rightarrow$ for each classification group of pieces of data, and, from the determined position and pose, the controller 16 calculates an error with respect to a design position and a design pose of the display section 13 as an assembling error in the display section 13.

[Method for Estimating Assembling Error in Active Light Source 11]

Next, a method for calculating an amount of correction between an optical axis and a visual axis in which an assembling error in hardware is taken into consideration, is described in detail, where the active light source 11 is assembling-error-estimation-target hardware.

Fig. 14 is a flowchart illustrating a procedure of a method for calculating an amount of correction between an optical axis and a visual axis in which an assembling error in the active light source 11 is taken into consideration.

Note that, in essence, a processing procedure in this case is similar to the method for calculating an amount of correction between an optical axis and a visual axis in which an assembling error in the display section 13 is taken into consideration.

21. First, the controller 16 controls the display section 13 such that a firstly presented gaze point from among N presented gaze points is displayed at a specified position on the screen of the display section 13 (Step S301).

22. Next, using the image-capturing section 12, the controller 16 repeatedly captures, L times, an image of the eyeball 1 of a user who is gazing at the firstly presented gaze position, while changing at least one of a position or a pose of the active light source 11. Accordingly, the controller acquires L images (first images) of the eyeball that correspond to the firstly presented gaze position (Step S302). The value of L is limited due to a speed of the information processing device 1, but is favorably the largest possible number for a reason of accuracy.

Using the image-capturing section 12, the controller 16 captures an image of the eyeball 1 of the user while changing at least one of a position or a pose of the active light source 11, and acquires position-and-pose information related to a position and a pose of the active light source 11 in a virtual space or in a real space. The position-and-pose information may be considered information depending on a change in at least one of the position or the pose of the active light source 11, the information including first position-and-pose information and second position-and-pose information that are different from each other. Then, the controller 16 associates the position-and-pose information regarding a position and a pose of the active light source 11 with an image (first image) of the eyeball that is captured in a state in which the active light source 11 is in the pose in the position, and saves them in the storage 14.

23. With respect to each of the L captured images, the controller 16 estimates a three-dimensional position $P_e{}^n$ of the center of a corneal curvature, and estimates a visual-axis vector $V_{vis}{}^n\rightarrow$ that connects the three-dimensional

position $P_e^n$ of the center of a corneal curvature with a three-dimensional position $P_{gt}^n$ of a presented gaze point (Step S303). Note that, since the three-dimensional position $P_e^n$ of the center of a corneal curvature does not depend on a position or a pose of the active light source 11, it is sufficient if the three-dimensional position $P_e^n$ of the center of a corneal curvature is estimated once for one presented gaze point.

24. With respect to each of the L captured images, the controller 16 estimates a three-dimensional position of a center of a pupil, and estimates an optical-axis vector $V_{opt}^n \rightarrow$ that connects the three-dimensional position of the center of a pupil with the three-dimensional position $P_e^n$ of the center of a corneal curvature (Step S304). Here, it is sufficient if the optical-axis vector $V_{opt}^n \rightarrow$ is estimated once for one presented gaze point.

25. The controller 16 saves, in the RAM 14 and as line-of-sight-calibration data, the estimated visual-axis vector $V_{vis}^n \rightarrow$, the estimated optical-axis vector $V_{opt}^n \rightarrow$, and the estimated three-dimensional position $P_e^n$ of the center of a corneal curvature described above (Step S305).

As a result of performing the processes of Step S302 to Step S305 described above, the controller 16 acquires L pieces of line-of-sight-calibration data for one presented gaze point.

26. Next, the controller 16 determines whether all of the $(N \times L)$ pieces of line-of-sight-calibration data for the N presented gaze points have been acquired (Step S306). When a piece of line-of-sight-calibration data to be acquired remains (NO in Step S306), the controller 16 returns to the process of Step S301. Then, for example, the controller 16 moves the firstly presented gaze point to a specified position (a second position) on the screen of the display section 13, and controls the display section 13 to display a secondly presented gaze point (a second presented gaze point).

When the secondly presented gaze point or a presented gaze point subsequent to the secondly presented gaze point is presented, the controller 16 changes the position and the pose of the image-capturing section 12 (in a virtual space or in a real space) by a change method similar to the method performed when the firstly presented gaze point is presented.

27. When all of the $(L \times N)$ pieces of line-of-sight-calibration data have been acquired (YES in Step S306), the controller 16 estimates an assembling error in the active light source 11 as indicated below (Step S307).

The controller 16 calculates differences between an optical-axis vector $V_{opt}^n \rightarrow$ and a visual-axis vector $V_{vis}^n \rightarrow$ with respect to the $(L \times N)$ respective acquired pieces of line-of-sight-calibration data. Using the pieces of position-and-pose information (pieces of first position-and-pose information and pieces of second position-and-pose information) associated with the respective pieces of line-of-sight-calibration data, the controller 16 classifies, by the same position and the same pose of the active light source 11, the differences between an optical-axis vector $V_{opt}^n \rightarrow$ and a visual-axis vector $V_{vis}^n \rightarrow$ that are calculated with respect to the $(L \times N)$ respective pieces of line-of-sight-calibration data.

Consequently, the $(L \times N)$ pieces of line-of-sight-calibration data are classified into L groups each including N pieces of line-of-sight-calibration data. The N pieces of line-of-sight-calibration data in each of the L groups created by the classification, are pieces of data acquired in a state in which the active light source 11 is in the same pose in the same position (in a virtual space or in a real space).

The controller 16 determines a position and a pose of the active light source 11 having a minimum variance of differences between an optical-axis vector $V_{opt}^n \rightarrow$ and a visual-axis vector $V_{vis}^n \rightarrow$ for each classification group of pieces of data, and, from the determined position and pose, the controller 16 calculates an error with respect to a design position and a design pose of the active light source 11 as an assembling error in the active light source 11.

Note that, when the information processing device 1 includes a plurality of active light sources 11, the processes described above are performed on each of the active light sources 11, and an assembling error is obtained for each active light source 11.

[Additional Notes]

(Example of Method for Estimating Three-Dimensional Position of Center of Corneal Curvature)

Fig. 15 illustrates an outline of a method for estimating a three-dimensional position of a center of a corneal curvature in a sensor-origin coordinate system.

In this example, the case in which there are two active light sources (a first active light source 11A and a second active light source 11B) is described.

This method is a method for estimating, using an image (a Purkinje image) 23, a three-dimensional position of a center 24 of a corneal curvature in a sensor-origin coordinate system, the image 23 including two bright spots respectively formed by infrared light 25A and infrared light 25B being reflected off the surface of a cornea 22 of an eyeball 1, the infrared light 25A and the infrared light 25B being respectively emitted from the two active light sources (the first active light source 11A and the second active light source 11B), that is, from different positions at different angles.

When a three-dimensional position of the first active light source 11A in the sensor coordinate system is $P_{L1}$, a three-dimensional position of the second active light source 11B in the sensor coordinate system is $P_{L2}$, a position of a center of a pupil in the image 23 captured by the image-capturing section 12 is $i_{cornea}$, a position of a reflection bright spot 21A of the first active light source 11A in the image 23 captured by the image-capturing section 12 is $i_{glints1}$, and a position of a reflection bright spot 21B of the second active light source 11B in the image 23 captured by the image-capturing section 12 is $i_{glints2}$, a three-dimensional position $p_{cornea}$ of the center 24 of a corneal curvature in the sensor-origin coordinate system is obtained using the following:

$$p_{cornea} = F_{cornea}(i_{cornea}, i_{glints1}, i_{glints2}, P_{L1}, P_{L2}).$$

$F_{cornea}$ is a function for obtaining $p_{cornea}$ using $i_{cornea}$, $i_{glints1}$, $i_{glints2}$, $P_{L1}$, and $P_{L2}$ as parameters.

(Example of Method for Estimating Three-Dimensional Position of Pupil)

Fig. 16 illustrates an outline of a method for estimating a three-dimensional position of a center 27 of a pupil.

When a three-dimensional position of the center 24 of a corneal curvature in the sensor-origin coordinate system is $p_{cornea}$, and a three-dimensional position of a center 28 of a pupil in the image 23 captured by the image-capturing section 12 is $i_{pupil}$, a three-dimensional position $p_{pupil}$ of the center 27 of a pupil in a world coordinate system is obtained using the following:

$$p_{pupil} = F_{pupil}(i_{pupil}, p_{cornea}).$$

Here, $F_{pupil}$ is a function for obtaining $p_{pupil}$ using $i_{pupil}$ and $p_{cornea}$ as parameters.

<Various Modifications>

(Configuration Without Active Light Source 11)

In the embodiment described above, the example of detecting a line of sight using infrared radiation emitted by the active light source 11, has been described, but the configuration is not limited to this. For example, there also exists a method for detecting and tracking a line of sight only using an image captured by the image-capturing section 12 without infrared radiation being irradiated. In this case, there is no need for the active light source 11. Fig. 17 illustrates a hardware configuration of the information processing device including a line-of-sight detection device that does not include the active light source.

(Cloud Configuration)

In the embodiment described above, the example in which the controller 16 included in the information processing device 100 performs arithmetic processing for estimating a line of sight, has been described, but a controller of a server may receive, from the information processing device 100 and through a network, each piece of data (such as captured-image data and hardware-design data) necessary to perform arithmetic processing for a line-of-sight calibration and a line-of-sight estimation, may perform processing for estimating a line of sight, and may transmit a result of it to the information processing device 100.

(Other Examples of Information Processing Device 100)

The head-mounted display has been described above as an example of the information processing device 100. On the other hand, the information processing device 100 may also be applied to various wearable devices other than a head-mounted display, or to a head-up display. Typically, the present technology is applicable to any device that detects a line of sight of a user and displays an image on a display section.

In order to measure a position and a pose of the information processing device 100 in a utilized space, the information processing device 100 may include one of an inside-out camera, a sensor (such as a gyroscope, an acceleration sensor, a geomagnetic sensor, a ultrasonic sensor, and an atmospheric-pressure sensor), an outside-in camera placed in a real space (in the utilized space) separately from the information processing device 100, and a sensor (such as an optical position tracker) placed in the real space separately from the information processing device, or a combination of two or more thereof.

Note that the inside-out camera may be considered a camera that is provided inside or on the information processing device and is used to track the position of the information processing device. Further, the outside-in camera may be considered a camera that is provided in a real space separately from the information processing device 100 and is used to track the position of the information processing device.

When the information processing device 100 is provided with a sensor that measures a position and a pose in a real space, it is possible to display, to a user, a presented gaze point situated at a fixed position in the space. Here, the background visually confirmed by the user may be an image of the real space (including a captured image of the real space), or a virtual image. In other words, the presented gaze point may be a so-called virtual object (an AR object or a VR object). When a position and a pose of a device are changed while causing a user to gaze at the presented gaze point, the direction of a line of sight of the user, viewed from a camera that captures an image of an eyeball of the user, varies relatively, and this results in providing the same effect as in the case of causing the user to gaze at a plurality of presented gaze points. In other words, the plurality of presented gaze points may be a virtual object situated at a fixed position in a space using pieces of position-and-pose information related to a position and a pose, the plurality of presented gaze points being associated with the respective pieces of position-and-pose information by the information processing device.

Here, for example, since an interpupillary distance (IPD) differs depending on a user, the adjustment section may be a mechanism for adjusting the position of the display section 13 according to the IPD of a user. For example, the adjustment section is a mechanism that adjusts how the things look on the screen of the display section 13, by adjusting the position of an eyepiece according to the IPD. Further, when the eyepiece is arranged on a single unit together with the image-capturing section 12 and the light source 11, the adjustment section can perform adjustment to an optimal position for capturing an image of an eyeball depending on the IPD, by adjusting the position and the pose of the unit. The controller 16 may acquire position-and-pose information depending on the adjustment related to the IPD. Further, the controller 16 may control the driving of the adjustment section according to a result of the correction described above.

Regarding a mechanism other than the IPD-adjustment mechanism described above, the information processing device 100 may include, as the adjustment section, a guide mechanism for adjusting the position and the pose of hardware (the image-capturing section 12, the display section 13, and the light source 11). In this case, at least one sensor that acquires position-and-pose information related to a position and a pose of the hardware of the information processing device 100, is provided together with the guide mechanism. This results in the controller 16 acquiring position-and-pose information related to a position and a pose of the hardware of the information processing device 100. Here, for example, the guide mechanism may be considered a mechanical mechanism that is capable of selecting a position and a pose from among a plurality of predetermined positions and poses, and performing fixation. Further, for example, the sensor may include at least one of a gyroscope, an acceleration sensor, a geomagnetic sensor, an ultrasonic sensor, an atmospheric-pressure sensor, or an external sensor placed in the environment (such as an outside-in camera).

When the information processing device 100 includes the adjustment section adjusting a position and a pose of hardware, a line-of-sight calibration may be performed simultaneously with the adjustment of a position and a pose of hardware being performed. In this case, a presented gaze point is adjusted such that a line of sight of a user is oriented toward any point fixed in a real space, the presented gaze point is displayed on the display section 13, and then an image of an eyeball of the user is captured. This makes it possible to acquire line-of-sight-calibration data for which an image of an eyeball of a user is captured in a state in which the hardware is in different poses in different positions, in which a line of sight of the user is oriented toward a point in a real space. Regarding the line-of-sight-calibration data, a position and a pose of hardware having a minimum variance of (or a minimum difference between) differences between an optical-axis vector and a visual-axis vector, reduces a calculation load when a position and a pose of hardware is estimated that has a minimum variance of (or a minimum difference between) differences between an optical-axis vector and a visual-axis vector, the minimum variance (or the minimum difference) being from among variances of (or differences between) differences between an optical-axis vector and a visual-axis vector, the variances (or the differences) corresponding to respective presented gaze points of a plurality of presented gaze points.

For example, by using, as an initial value, the above-described position and pose of hardware having a minimum variance of (or a minimum difference between) differences between an optical-axis vector and a visual-axis vector, it becomes possible to reduce a calculation load when a position and a pose of hardware is determined in terms of minimization.

Note that the present technology may also take the following configurations.

(1) An information processing device including
a controller configured to

acquire first position-and-pose information related to a position and a pose of hardware that is used to calibrate a line of sight, and second position-and-pose information related to a position and a pose of the hardware, the second position-and-pose information being different from the first position-and-pose information,
control a display section to display a plurality of presented gaze points including a first presented gaze point and a second presented gaze point, the first presented gaze point being presented at a first position, the second presented gaze point being presented at a second position different from the first position,

control an image-capturing section to acquire a first image of an eyeball of a user gazing at the first presented gaze point, and a first image of the eyeball of the user gazing at the second presented gaze point,

generate a plurality of pieces of calibration data using the first position-and-pose information, the second position-and-pose information, and the first images, and

perform a correction related to an optical-axis vector and a visual-axis vector with respect to the eyeball of the user, using the plurality of pieces of calibration data.

(2) The information processing device according to (1), in which
the hardware is at least one of the image-capturing section, the display section, or a light source that irradiates the eyeball of the user with light.

(3) The information processing device according to (1) or (2), in which
the plurality of pieces of calibration data includes a first piece of calibration data based on the first position-and-pose information, and a second piece of calibration data based on the second position-and-pose information, the first piece of calibration data including information related to the optical-axis vector and the visual-axis vector, the second piece of calibration data including information related to the optical-axis vector and the visual-axis vector, and the controller is configured to perform the correction using a piece of calibration data having a smaller difference between a certain difference between an optical-axis vector and a visual-axis vector, and another difference between an optical-axis vector and a visual-axis vector, the piece of calibration data being the first piece of calibration data or the second piece of calibration data, the certain difference corresponding to the first presented gaze point, the other difference corresponding to the second presented gaze point.

(4) The information processing device according to (3), in which
the controller is configured to perform the correction using a piece of calibration data having a smaller variance of differences between an optical-axis vector and a visual-axis vector, the piece of calibration data being the first piece of calibration data or the second piece of calibration data, the differences corresponding to respective presented gaze points of the plurality of presented gaze points.

(5) The information processing device according to any one of (1) to (4), in which
the first presented gaze point is a presented gaze point different from the second presented gaze point.

(6) The information processing device according any one of (1) to (5), in which
the second presented gaze point is the first presented gaze point displaced to the second position from the first position.

(7) The information processing device according any one of (1) to (5), in which
the first presented gaze point and the second presented gaze point are a virtual object situated at a fixed position in a space using pieces of position-and-pose information related to a position and a pose, the first presented gaze point and the second presented gaze point being associated with the respective pieces of position-and-pose information by the information processing device.

(8) The information processing device according to (3), in which the controller is configured to

control the image-capturing section to acquire a second image of the eyeball of the user,

estimate the optical-axis vector using the second image,

estimate the visual-axis vector by performing correction with respect to the estimated optical-axis vector according to a correction amount, and

calculate the correction amount using the piece of calibration data having a smaller difference between the certain difference and the other difference, the piece of calibration data being the first piece of calibration data or the second piece of calibration data.

(9) The information processing device according to (8), in which
the controller is configured to calculate the correction amount using a similarity between the estimated optical-axis vector and the optical-axis vector included in the piece of calibration data having a smaller difference between the certain difference and the other difference, the piece of calibration data being the first piece of calibration data or the second piece of calibration data.

(10) The information processing device according to (9), in which
the first piece of calibration data and the second piece of calibration data each further include at least one of information regarding a three-dimensional position of a center of a corneal curvature of the eyeball of the user, or information regarding a three-dimensional position of a center of a pupil or a center of an eyeball rotation of the eyeball of the user, and

the controller is configured to

estimate, using the second image, at least one of the three-dimensional position of the center of a corneal curvature, or the three-dimensional position of the center of a pupil or the center of an eyeball rotation, and calculate the correction amount using at least one of a similarity between the estimated three-dimensional position of the center of a corneal curvature and another three-dimensional position of the center of a corneal curvature, or a similarity between the estimated three-dimensional position of the center of a pupil or the center of an eyeball rotation, and another three-dimensional position of the center of a pupil or the center of an eyeball rotation, the other three-dimensional position of the center of a corneal curvature and the other three-dimensional position of the center of a pupil or the center of an eyeball rotation being included in the piece of calibration data having a smaller difference between the certain difference and the other difference, the piece of calibration data being the first piece of calibration data or the second piece of calibration data.

(11) The information processing device according to any one of (1) to (10), in which
the controller is configured to acquire the first position-and-pose information and the second position-and-pose information depending on a movement of the hardware in a real space.

(12) The information processing device according to (11), in which
the controller is configured to control the display section to display guidance indicating a method for moving the hardware.

(13) The information processing device according to (12), in which
the controller is configured to acquire the first position-and-pose information and the second position-and-pose information after the guidance is displayed.

(14) The information processing device according to (11), further including an adjustment section configured to adjust at least one of a position or a pose of the hardware, in which
the controller is configured to acquire the first position-and-pose information and the second position-and-pose information depending on the adjustment.

(15) The information processing device according to (14), in which
the adjustment section is configured to automatically perform the adjustment using a drive section, and
the controller drives the adjustment section according to a result of the correction.

(16) The information processing device according to (14), in which
the adjustment section is configured to adjust an interpupillary distance of a head-mounted display or a head-up display, and
the controller is configured to acquire the first position-and-pose information and the second position-and-pose information depending on the adjustment of the interpupillary distance.

(17) The information processing device according to (14), in which
the adjustment section is a mechanism for a head-mounted display or a head-up display that is capable of manually performing the adjustment.

(18) The information processing device according to any one of (1) to (17), in which
the information processing device is a head-mounted display that further includes the display section and the image-capturing section.

(19) An information processing method including:

acquiring first position-and-pose information related to a position and a pose of hardware that is used to calibrate a line of sight, and second position-and-pose information related to a position and a pose of the hardware, the second position-and-pose information being different from the first position-and-pose information;
controlling a display section to display a plurality of presented gaze points including a first presented gaze point and a second presented gaze point, the first presented gaze point being presented at a first position, the second presented gaze point being presented at a second position different from the first position;
controlling an image-capturing section to acquire a first image of an eyeball of a user gazing at the first presented gaze point, and a first image of the eyeball of the user gazing at the second presented gaze point;
generating a plurality of pieces of calibration data using the first position-and-pose information, the second position-and-pose information, and the first images; and
performing a correction related to an optical-axis vector and a visual-axis vector with respect to the eyeball of the user, using the plurality of pieces of calibration data.

(20) A program that causes a computer to perform a process including:

acquiring first position-and-pose information related to a position and a pose of hardware that is used to calibrate a line of sight, and second position-and-pose information related to a position and a pose of the hardware, the second position-and-pose information being different from the first position-and-pose information;

controlling a display section to display a plurality of presented gaze points including a first presented gaze point and a second presented gaze point, the first presented gaze point being presented at a first position, the second presented gaze point being presented at a second position different from the first position;
controlling an image-capturing section to acquire a first image of an eyeball of a user gazing at the first presented gaze point, and a first image of the eyeball of the user gazing at the second presented gaze point;
generating a plurality of pieces of calibration data using the first position-and-pose information, the second position-and-pose information, and the first images; and
performing a correction related to an optical-axis vector and a visual-axis vector with respect to the eyeball of the user, using the plurality of pieces of calibration data.

Reference Signs List

| 10 | line-of-sight detection device |
|---|---|
| 11 | active light source |
| 12 | image-capturing section |
| 13 | display section |
| 14 | storage |
| 16 | controller |
| 100 | information processing device |

**Claims**

1. An information processing device comprising
   a controller configured to

   acquire first position-and-pose information related to a position and a pose of hardware that is used to calibrate a line of sight, and second position-and-pose information related to a position and a pose of the hardware, the second position-and-pose information being different from the first position-and-pose information,
   control a display section to display a plurality of presented gaze points including a first presented gaze point and a second presented gaze point, the first presented gaze point being presented at a first position, the second presented gaze point being presented at a second position different from the first position,
   control an image-capturing section to acquire a first image of an eyeball of a user gazing at the first presented gaze point, and a first image of the eyeball of the user gazing at the second presented gaze point,
   generate a plurality of pieces of calibration data using the first position-and-pose information, the second position-and-pose information, and the first images, and
   perform a correction related to an optical-axis vector and a visual-axis vector with respect to the eyeball of the user, using the plurality of pieces of calibration data.

2. The information processing device according to claim 1, wherein
   the hardware is at least one of the image-capturing section, the display section, or a light source that irradiates the eyeball of the user with light.

3. The information processing device according to claim 1, wherein
   the plurality of pieces of calibration data includes a first piece of calibration data based on the first position-and-pose information, and a second piece of calibration data based on the second position-and-pose information, the first piece of calibration data including information related to the optical-axis vector and the visual-axis vector, the second piece of calibration data including information related to the optical-axis vector and the visual-axis vector, and the controller is configured to perform the correction using a piece of calibration data having a smaller difference between a certain difference between an optical-axis vector and a visual-axis vector, and another difference between an optical-axis vector and a visual-axis vector, the piece of calibration data being the first piece of calibration data or the second piece of calibration data, the certain difference corresponding to the first presented gaze point, the other difference corresponding to the second presented gaze point.

4. The information processing device according to claim 3, wherein
   the controller is configured to perform the correction using a piece of calibration data having a smaller variance of differences between an optical-axis vector and a visual-axis vector, the piece of calibration data being the first piece of calibration data or the second piece of calibration data, the differences corresponding to respective presented gaze points of the plurality of presented gaze points.

5. The information processing device according to claim 1, wherein
the first presented gaze point is a presented gaze point different from the second presented gaze point.

6. The information processing device according to claim 1, wherein
the second presented gaze point is the first presented gaze point displaced to the second position from the first position.

7. The information processing device according to claim 1, wherein
the first presented gaze point and the second presented gaze point are a virtual object situated at a fixed position in a space using pieces of position-and-pose information related to a position and a pose, the first presented gaze point and the second presented gaze point being associated with the respective pieces of position-and-pose information by the information processing device.

8. The information processing device according to claim 3, wherein the controller is configured to

control the image-capturing section to acquire a second image of the eyeball of the user,
estimate the optical-axis vector using the second image, estimate the visual-axis vector by performing correction with respect to the estimated optical-axis vector according to a correction amount, and
calculate the correction amount using the piece of calibration data having a smaller difference between the certain difference and the other difference, the piece of calibration data being the first piece of calibration data or the second piece of calibration data.

9. The information processing device according to claim 8, wherein
the controller is configured to calculate the correction amount using a similarity between the estimated optical-axis vector and the optical-axis vector included in the piece of calibration data having a smaller difference between the certain difference and the other difference, the piece of calibration data being the first piece of calibration data or the second piece of calibration data.

10. The information processing device according to claim 9, wherein
the first piece of calibration data and the second piece of calibration data each further include at least one of information regarding a three-dimensional position of a center of a corneal curvature of the eyeball of the user, or information regarding a three-dimensional position of a center of a pupil or a center of an eyeball rotation of the eyeball of the user, and
the controller is configured to

estimate, using the second image, at least one of the three-dimensional position of the center of a corneal curvature, or the three-dimensional position of the center of a pupil or the center of an eyeball rotation, and
calculate the correction amount using at least one of a similarity between the estimated three-dimensional position of the center of a corneal curvature and another three-dimensional position of the center of a corneal curvature, or a similarity between the estimated three-dimensional position of the center of a pupil or the center of an eyeball rotation, and another three-dimensional position of the center of a pupil or the center of an eyeball rotation, the other three-dimensional position of the center of a corneal curvature and the other three-dimensional position of the center of a pupil or the center of an eyeball rotation being included in the piece of calibration data having a smaller difference between the certain difference and the other difference, the piece of calibration data being the first piece of calibration data or the second piece of calibration data.

11. The information processing device according to claim 1, wherein
the controller is configured to acquire the first position-and-pose information and the second position-and-pose information depending on a movement of the hardware in a real space.

12. The information processing device according to claim 11, wherein
the controller is configured to control the display section to display guidance indicating a method for moving the hardware.

13. The information processing device according to claim 12, wherein
the controller is configured to acquire the first position-and-pose information and the second position-and-pose information after the guidance is displayed.

14. The information processing device according to claim 11, further comprising an adjustment section configured to adjust at least one of a position or a pose of the hardware, wherein
the controller is configured to acquire the first position-and-pose information and the second position-and-pose information depending on the adjustment.

15. The information processing device according to claim 14, wherein
the adjustment section is configured to automatically perform the adjustment using a drive section, and
the controller drives the adjustment section according to a result of the correction.

16. The information processing device according to claim 14, wherein
the adjustment section is configured to adjust an interpupillary distance of a head-mounted display or a head-up display, and
the controller is configured to acquire the first position-and-pose information and the second position-and-pose information depending on the adjustment of the interpupillary distance.

17. The information processing device according to claim 14, wherein
the adjustment section is a mechanism for a head-mounted display or a head-up display that is capable of manually performing the adjustment.

18. The information processing device according to claim 1, wherein
the information processing device is a head-mounted display that further includes the display section and the image-capturing section.

19. An information processing method comprising:

acquiring first position-and-pose information related to a position and a pose of hardware that is used to calibrate a line of sight, and second position-and-pose information related to a position and a pose of the hardware, the second position-and-pose information being different from the first position-and-pose information;
controlling a display section to display a plurality of presented gaze points including a first presented gaze point and a second presented gaze point, the first presented gaze point being presented at a first position, the second presented gaze point being presented at a second position different from the first position;
controlling an image-capturing section to acquire a first image of an eyeball of a user gazing at the first presented gaze point, and a first image of the eyeball of the user gazing at the second presented gaze point;
generating a plurality of pieces of calibration data using the first position-and-pose information, the second position-and-pose information, and the first images; and
performing a correction related to an optical-axis vector and a visual-axis vector with respect to the eyeball of the user, using the plurality of pieces of calibration data.

20. A program that causes a computer to perform a process comprising:

acquiring first position-and-pose information related to a position and a pose of hardware that is used to calibrate a line of sight, and second position-and-pose information related to a position and a pose of the hardware, the second position-and-pose information being different from the first position-and-pose information;
controlling a display section to display a plurality of presented gaze points including a first presented gaze point and a second presented gaze point, the first presented gaze point being presented at a first position, the second presented gaze point being presented at a second position different from the first position;
controlling an image-capturing section to acquire a first image of an eyeball of a user gazing at the first presented gaze point, and a first image of the eyeball of the user gazing at the second presented gaze point;
generating a plurality of pieces of calibration data using the first position-and-pose information, the second position-and-pose information, and the first images; and
performing a correction related to an optical-axis vector and a visual-axis vector with respect to the eyeball of the user, using the plurality of pieces of calibration data.

U

100

FIG.1

10

11

1

$\overrightarrow{V_{opt}}$ Optical-axis vector

$P_{gt}^{n}$

$P_{e}^{n}$

$\overrightarrow{V_{vis}}$ Visual-axis vector

13

12

FIG.2

Display section — 13

Image-capturing section — 12

Active light source — 11

Storage

Controller

15

16

FIG.3

Actual (with assembling error)

# FIG.4A

Plane of display

13

Corrected gaze point

y

12

Calculated (no assembling error)

# FIG.4B

Actual (with assembling error)

FIG.5A

FIG.5B

# FIG.6A

Plane of display

13

$\overrightarrow{V_{vis}}$

$\overrightarrow{V_{opt}}$ ● Presented gaze point

y

θr

1

12

$P_e$

Actual (with assembling error)

# FIG.6B

Plane of display

13

$\overrightarrow{V_{vis}}$

$\overrightarrow{V_{opt}}$

● Presented gaze point
● Corrected gaze
point

y

θr θa

1

12

$P_e$

Calculated (no assembling error)

## FIG.7A

Actual (with assembling error)

## FIG.7B

Calculated (no assembling error)

## FIG.8A

$\overrightarrow{V_{vis}}$

$\overrightarrow{V_{opt}}$

$\theta$ r

$P_e$

Presented gaze point

1

12

13
Plane of display

Actual (with assembling error)

## FIG.8B

Corrected gaze point

12

y

$\theta$ b

$\overrightarrow{V_{vis}}$

$\theta$ r

$\overrightarrow{V_{opt}}$

$P_e$

1

13
Plane of display

Calculated (no assembling error)

FIG.9A

FIG.9B

Actual (with assembling error)

FIG.10A

Plane of display

13

Corrected gaze point 1   y

12

$\overrightarrow{V_{vis}^1}$

$\overrightarrow{V_{opt}^1}$   $\theta\,r$
$\theta\,c1$   $\overrightarrow{V_{opt}^2}$   $\overrightarrow{V_{vis}^2}$

Corrected gaze point 2

$\theta\,r$
$\theta\,c2$   $\overrightarrow{V_{opt}^3}$   $\overrightarrow{V_{vis}^3}$

1   $\theta\,r$
$\theta\,c3$   Corrected gaze point 3

$P_e^1$   $P_e^2$
$P_e^3$

Calculated (no assembling error)

FIG.10B

Present (nth) presented gaze point — S101

Capture image of eyeball — S102

Estimate optical-axis start point $P_e^n$ and visual-axis vector $\overrightarrow{V_{vis}^n}$ — S103

Estimate optical-axis vector $\overrightarrow{V_{opt}^n}$ — S104

Record $\overrightarrow{V_{vis}^n}$, $P_e^n$, and $\overrightarrow{V_{opt}^n}$ as line-of-sight-calibration data — S105

S106 — Have all of the (N×L) pieces of line-of-sight-calibration data for N presented gaze points been acquired? — No

Yes

Estimate assembling error in camera — S107

S108 — Calculate amount of correction between optical axis and visual axis using optical-axis start point $P_e$ and optical-axis vector $\overrightarrow{V_{opt}}$ with respect to line-of-sight-estimation-target eyeball, line-of-sight-calibration data, and assembling error in camera

FIG.11

FIG.12

Present (nth) presented gaze point — S201

Capture image of eyeball — S202

S203

Estimate optical-axis start point $P_e^n$ and visual-axis vector $\overrightarrow{V_{vis}^n}$
(Optical-axis start point $P_e^n$ is estimated only once for one
presented gaze point)

Estimate optical-axis vector $\overrightarrow{V_{opt}^n}$
(Only once for one presented gaze point) — S204

Record $\overrightarrow{V_{vis}^n}$, $P_e^n$, and $\overrightarrow{V_{opt}^n}$ as
line-of-sight-calibration data — S205

S206
Have all of the (N×L)
pieces of line-of-sight-calibration data
for N presented gaze points been
acquired? — No

Yes

Estimate assembling error in display — S207

S208
Calculate amount of correction between optical axis and visual
axis using optical-axis start point $P_e$ and optical-axis vector
$\overrightarrow{V_{opt}}$ with respect to line-of-sight-estimation-target eyeball,
line-of-sight-calibration data, and assembling error in display

FIG.13

●

Present (nth) presented gaze point — S301

Capture image of eyeball — S302

S303

Estimate optical-axis start point $P_e^n$ and visual-axis vector $\overrightarrow{V_{vis}^n}$
(Optical-axis start point $P_e^n$ is estimated only once for one
presented gaze point)

Estimate optical-axis vector $\overrightarrow{V_{opt}^n}$
(Only once for one presented gaze point) — S304

Record $\overrightarrow{V_{vis}^n}$, $P_e^n$, and $\overrightarrow{V_{opt}^n}$ as
line-of-sight-calibration data — S305

S306

Have all of the (N×L)
pieces of line-of-sight-calibration data
for N presented gaze points been
acquired? — No

Yes

Estimate assembling error in active light source — S307

S308

Calculate amount of correction between optical axis and visual
axis using optical-axis start point $P_e$ and optical-axis vector
$\overrightarrow{V_{opt}}$ with respect to line-of-sight-estimation-target eyeball,
line-of-sight-calibration data, and assembling error in active
light source

FIG.14

FIG.15

EP 3 657 299 A1

28  23 Infrared-image of eyeball

12

13

1 Eyeball

22 Cornea

26
27

24 Center of
corneal curvature

FIG.16

EP 3 657 299 A1

Display section —13

Image-capturing section —12

Storage

Controller

15

16

FIG.17

EP 3 657 299 A1

## INTERNATIONAL SEARCH REPORT

| International application No. |
|---|
| PCT/JP2018/025843 |

### A. CLASSIFICATION OF SUBJECT MATTER
Int.Cl. G06F3/01(2006.01)i, A61B3/113(2006.01)i, G02B27/02(2006.01)i,
        G06F3/038(2013.01)i, G09G5/00(2006.01)i, H04N5/64(2006.01)i

According to International Patent Classification (IPC) or to both national classification and IPC

### B. FIELDS SEARCHED

Minimum documentation searched (classification system followed by classification symbols)
Int.Cl. G06F3/01, A61B3/113, G02B27/02, G06F3/038, G09G5/00, H04N5/64

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2016/098406 A1 (SONY CORP.) 23 June 2016, paragraphs [0013]-[0087] & US 2017/0329400 A1, paragraphs [0038]-[0111] | 1-2, 5-7, 11-13, 18-20 |
| Y | & EP 3236338 A1 & CN 107003752 A & KR 10-2017-0095194 A | 14-17 |
| A | | 3-4, 8-10 |
| Y | JP 2016-536869 A (MAGIC LEAP, INC.) 24 November 2016, paragraphs [0029], [0048], [0059] & US 2015/0103306 A1, paragraphs [0058], [0077], [0088] & WO 2015/057994 A1 & KR 10-2016-0072197 A & CN 105829953 A | 14-17 |
| A | | 3-4,8-10 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 August 2018 (06.08.2018) | 14 August 2018 (14.08.2018) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

## REFERENCES CITED IN THE DESCRIPTION

**Patent documents cited in the description**

• JP 2009183473 A